# EUROPEAN PATENT APPLICATION

(11) **EP 1 106 701 A1**
(43) Date of publication of application: **13.06.2001**
(21) Application number: 99935112.5
(22) Date of filing: 09.08.1999
(51) Int. Cl.: C12P 21/08, C07K 16/40, C07K 16/18, C12Q 1/34, G01N 33/573, G01N 33/577, C12N 15/06

(54) **MONOCLONAL ANTIBODY AGAINST CANINE TRYPSIN**

(30) Priority: 10.08.1998 JP 23660998; 10.03.1999 JP 6399099
(71) Applicant: FUJI YAKUHIN KOGYO KABUSHIKI KAISHA, Takaoka-shi Toyama 933-8511 (JP)
(72) Inventor: FWARITANI, Takaki, Fuji Yakuhin Kogyo K.K., Takaoka-shi, Toyama 933-8511 (JP); ASHIDA, Yoshinori, Fuji Yakuhin Kogyo K.K., Takaoka-shi, Toyama 933-8511 (JP); YAMADA, Takatsugu, Hachioji-shi, Tokyo 192-0364 (JP)
(74) Representative: Helbing, Jörg, Dr. Dipl.-Chem.
(86) International application number: JP9904299
(87) International publication number: WO0009739

(57) **Abstract**

The in vivo dynamics of trypsin, in particular canine trypsin and/or canine trypsin-like immunoreactivity have attracted attention in analyzing the relation thereof with various diseases such as pancreatic diseases. Since trypsin occurs in various forms including two subclass forms, i.e., a cationic form and an anionic form, it is also required to assay separately individual species thereof. To satisfy the above requirements, methods for accurately quantitating, measuring or detecting trypsin. Monoclonal antibodies specifically immunoreactive to trypsin can be produced by the cell fusion wherein trypsin, in particular canine trypsin or a fragment having a specific amino acid sequence or neighborhood thereof, is used as an immunogen. By using as an assay reagent the monoclonal antibody thus obtained, trypsin and/or trypsin-like immunoreactivity present in various forms can be quickly and accurately assayed by, in particular, sandwich assays, etc. Moreover, the clinical meaning of the ratio of respective trypsin, etc. in diseases (acute pancreatitis, chronic pancreatitis, pancreatic cancer, renal insufficiency, exocrine pancreatic insufficiency, etc.) can be clarified thereby.

## Description

### BACKGROUND OF THE INVENTION

### 1. Technical Field of the Invention

The present invention relates to a monoclonal antibody (MAb) to canine trypsin or a substance related thereto, to an immunoassay for trypsin and/or trypsin-like immunoreactivity which comprises using the said MAb and to an immunoassay reagent for trypsin and/or trypsin-like immunoreactivity which comprises an effective amount of the said MAb. In another aspect, the present invention relates to a method for diagnosing a member selected from diseases and disorders including pancreas diseases such as acute pancreatitis, chronic pancreatitis, pancreatic cancer and exocrine pancreatic insufficiency, splanchnic diseases such as renal insufficiency, etc. which comprises using the said immunoassay reagent and/or the said immunoassay, and further to an agent for diagnosis thereof.

### 2. Description of Related Art

Trypsin is one of proteinases (proteolytic enzymes) occurring among various animals including human, dog, cat, bovine, horse, swine, sheep, goat, etc. and biosynthesized in a zymogen precursor, trypsinogen, by pancreatic acinous cells of the pancreas. Trypsinogen is secreted into the duodenum followed by conversion into the active form, trypsin, by the hydrolytic action of enterokinase or trypsin itself. Trypsin predominantly acts to digest dietary proteins in the intestine. Further, trypsin restrictively degrades other zymogen precursors including chymotrypsinogen, procarboxypeptidase, prophospholipase, etc. to produce active form enzymes. It has been known that human trypsinogen includes two subtypes, i.e., cationic form (cationic trypsinogen) and anionic form (anionic trypsinogen).

Trypsin, produced by pancreas only, is a highly organ-specific enzyme and an extremely specific enzyme among pancreatic enzymes. It is suggested that its in vivo change reflects the conditions of pancreas including for example pancreatic diseases, etc., whereby trypsin draws attention. Trypsin is normally present in an inactive form, trypsinogen, in blood but when diseases such as pancreatitis occurs the active form trypsin deviated from injured pancreatic cells is present in complexes coupled with inhibitors such as *α* 1-antitrypsin and *α* 2-macroglobulin. Therefore, blood trypsin is generally called TLI (trypsin-like immunoreactivity) in order to cover all species thereof.

With respect to this blood TLI, it has been known that blood TLI levels are elevated in human acute pancreatitis, pancreatic cancer and renal insufficiency while lowered in human exocrine pancreatic insufficiency and chronic pancreatitis (Immunoassays of Blood Enzymes and Clinical Applications Thereof, pp. 223-231, 1984, Health Publisher, Japan). It is also conducted that human pancreatitis is diagnosed by assaying the amount of urine trypsin (N. Engl. J. Med., 336, pp.1788-1793, 1997).

It has been reported that trypsin assays are based on measuring the enzymatic activity of trypsin by using a substrate such as benzoyl-L-arginine amide. However, in these techniques, there are problems that when blood trypsin is attempted to be assayed an accurate measurement cannot be carried out because trypsin exists even in those forms coupled with inhibitors such as α 1-antitrypsin and α 2-macroglobulin in blood as aforementioned.

To solve these drawbacks, immunological methods (immunoassays) have been developed for assaying trypsin. In the prior art, antibodies as used in these immunoassays have been prepared by separation from sera obtained from animals such as rabbits immunized with antigens (those thus prepared are polyclonal antibodies). These methods have problems that each antibody production requires a large amount of antigens, antibodies are produced in small yields, the resultant antibodies themselves are not homogeneous (for example, their titer is not always constant, and/or antibodies against diverse antigens are mixed together), etc. Therefore, it has been difficult to conduct more precise and accurate assays or highly reproducible assays thereby. Further, it has been impossible to selectively assay each individual trypsin species in various forms including two subtypes, cationic and anionic forms, in vivo (for example, in blood, etc.) thereby.

For human, with the aim of inexpensively obtaining more homogeneous antibodies to trypsin, a monoclonal antibody produced by a hybridoma obtained by utilization of cell fusion techniques has been developed. Thus it has been reported that MAb is produced which recognizes human anionic trypsin (N. Engl. J. Med., 336, pp.1788-1793, 1997).

In the veterinary field, blood TLI has been assayed using polyclonal antibodies, particularly in dog. It has been found that blood TLI rises at an intial stage of acute pancreatitis (Am. J. Vet. Res., Vol.50, No.5, pp.629-632, 1989) while it significantly decreases in exocrine pancreatic insufficiency (J. Am. Vet. Med. Assoc., 192, pp.195-201, 1988; J. Small Anim. Pract., 24, pp.583-588, 1983).

Canine trypsinogen includes two subtypes, cationic and anionic forms, whose gene has already been cloned. Canine cationic trypsinogen has an amino acid sequence of SEQ ID NO: 2 in the Sequence Listing while the anionic form has an amino acid sequence of SEQ ID NO: 1 in the Sequence Listing. For the amino acid sequences as illustrated in the Sequence Listing, a Met¹ to Ala¹⁵ sequence is considered as a transport signal sequence. Therefore, it is thought that mature canine cationic trypsinogen actually has 231 amino acid residues corresponding to a Phe¹⁶ to Asn²⁴⁶ sequence of SEQ ID NO: 2 in the Sequence Listing. Similarly, it is thought that mature canine anionic trypsinogen has 232 amino acid residues corresponding to a Thr¹⁶ to Ser²⁴⁷ sequence of SEQ ID NO: 1 in the Sequence Listing. By liberation of N-terminal 8 residues thereof, canine cationic trypsin comes to 223 amino acid residues in an active form and active canine anionic trypsin is also formed with 224 amino acid residues (Mol. Cell. Biol., 5, 2669-2676, 1985).

For human, various kits of radioimmunoassay (RIA), enzyme immunoassay (EIA), etc. are commercially available wherein a polyclonal antibody is combined with a monoclonal antibody. Further, there is a kit for assaying urine TLI, said kit not only containing a monoclonal antibody capable of recognizing human anionic trypsin but also using the lateral flow method. However, it is impossible to. assay canine TLI by using this kit.

There is an RIA kit for assaying canine trypsin wherein rabbit is immunized with cationic trypsin purified from dog pancreas and the resultant polyclonal antibody is used (J. Am. Vet. Med. Assoc., 192, pp.195-201, 1988; J. Small Anim. Pract., 24, pp.583-588, 1983, etc.). However, it is impossible to assay, with such a kit, not only various forms of trypsin, including its inactive form, i.e., trypsinogen, its free active form, i.e., trypsin (including their two subtypes, i.e., the cationic and anionic forms), those species coupled with inhibitors such as *α* 1-antitrypsin and *α* 2-macroglobulin, etc., but also each individual thereof and each occurrence ratio thereof.

It is believed that trypsin, which is present in various forms in vivo as aforementioned, dynamically varies for the ratio of each individual form, etc. depending on diseases. If we can grasp dynamic changes in such a ratio, we may utilize them in the diagnosis of various diseases. However, the assay system using MAb which has been developed for human cannot enable canine trypsin to be assayed at all. Furthermore, it is impossible to assay the ratio, etc. of trypsin present in the aforementioned various forms even with assay systems for canine trypsin using polyclonal antibodies. Thus, it has been impossible to examine in detail the presence ratio, etc. of dynamic TLI dependent on diseases in the prior art.

Further, there has been a demand to construct a more sensitive and reliable trypsin assay system even without using radioactive labels which not only are required to pay special attention to safety and handling but also have serious problems in view of waste after assay. Therefore, it is great benefit in light of diagnosing diseases and disorders to assay selectively trypsin including each individual form and/or their respective ratios by quick and accurate, further simple, convenient and riskless methods.

Accordingly, there has been a demand for assay systems capable of measuring and/or detecting canine trypsin with higher selectivity.

### SUMMARY OF THE INVENTION

In order to solve such problems, the present inventors have made an extensive research and study. As a result, the present inventors have succeeded in producing monoclonal antibodies immunologically reactive to canine trypsin, and further in developing a highly selective assay system for dog trypsin wherein the said anti-canine trypsin monoclonal antibody thus prepared is employed.

Thus, the present invention provides:
(1) a monoclonal antibody against canine trypsin or a canine trypsin-related substance;
(2) the monoclonal antibody according to the above (1) which is an antibody to at least one member selected from the group consisting of canine cationic trypsinogen, canine anionic trypsinogen, canine cationic trypsin, canine anionic trypsin and canine trypsin-related substances derived therefrom;
(3) the monoclonal antibody according to the above (1) which is an antibody to at least one member selected from the group consisting of (a) a protein of SEQ ID NO: 1 or SEQ ID NO: 2 in the Sequence Listing or a salt thereof and (b) a peptide fragment thereof or a salt thereof;
(4) the monoclonal antibody according to any of the above (1) to (3) which is an antibody to at least one member selected from the group consisting of canine cationic trypsinogen, canine cationic trypsin, and canine cationic trypsin-related substance derived therefrom;
(5) the monoclonal antibody according to any of the above (1) to (3) which is an antibody to at least one member selected from the group consisting of
   (1) a Thr¹⁶ to Ser²⁴⁷ amino acid sequence of SEQ ID NO:1 in the Sequence Listing,
   (2) an Ile²⁴ to Ser²⁴⁷ amino acid sequence of SEQ ID NO:1 in the Sequence Listing,
   (3) a Phe¹⁶ to Asn²⁴⁶ amino acid sequence of SEQ ID NO:2 in the Sequence Listing, and
   (4) an Ile²⁴ to Asn²⁴⁶ amino acid sequence of SEQ ID NO:2 in the Sequence Listing;
(6) an immunoassay for trypsin and/or trypsin-like immunoreactivity (TLI) which comprises using an assay reagent containing the monoclonal antibody according to any of the above (1) to (5);
(7) the immunoassay according to the above (6) wherein the trypsin-like immunoreactivity (TLI) is a member selected from the group consisting of trypsinogen, trypsin and their complexes with inhibitors;
(8) an immunoassay reagent for trypsin and/or trypsin-like immunoreactivity (TLI) which comprises an effective amount of the monoclonal antibody according to any of the above (1) to (5); and
(9) the immunoassay reagent according to the above (8) wherein the trypsin-like immunoreactivity (TLI) is a member selected from the group consisting of trypsinogen, trypsin and their complexes with inhibitors.

In another aspect, the present invention provides:
(10) the antibody according to any of the above (1) to (5) which is a monoclonal antibody insolubilized on a solid phase;
(11) the antibody according to any of the above (1) to (5) which is a labeled monoclonal antibody;
(12) the antibody according to any of the above (1) to (5) which is a monoclonal antibody labeled with an enzyme or a metal particle; and
(13) the antibody according to any of the above (1) to (5) which is a labeled Fab' fragment from MAb.

In yet another aspect, the present invention provides:
(14) the immunoassay according to the above (6) wherein at least two antibodies according to any of the above (1) to (5) are used provided that (i) one of the antibodies is insolubilized on a solid phase and (ii) another is labeled;
(15) the immunoassay according to the above (6) which quantitatively assays canine trypsin or a canine trypsin-related substance;
(16) the immunoassay according to the above (6) or (15) wherein the sample to be assayed is selected from the group consisting of blood, serum, plasma, articular fluid, urine, saliva, stools, tissue extracts, cultured cell extracts and cell culture supernatants;
(17) a set of assay reagents for trypsin which comprises at least two reagents wherein (i) one of the reagents is an antibody insolubilized on a solid phase, said antibody being selected from the antibody according to any of the above (1) to (5) and (ii) another is a labeled antibody selected from the antibody according to any of the above (1) to (5), provided that said reagents are for the application to the immunoassay according to the above (6), (7) and (14) to (16);
(18) a hybridoma capable of producing a monoclonal antibody (MAb) to at least one member selected from the group consisting of (a) a protein of SEQ ID NO: 1 or SEQ ID NO: 2 in the Sequence Listing or a salt thereof and (b) a peptide fragment thereof or a salt thereof which is produced by fusing a splenic cell from an animal such as a mouse which is immunized with a member selected from the group consisting of (a) a protein of SEQ ID NO: 1 or SEQ ID NO: 2 in the Sequence Listing or a salt thereof and (b) a peptide fragment thereof or a salt thereof, with an immortalized cell including an animal myeloma cell such as a mouse myeloma cell; and
(19) a method for producing the hybridoma according to the above (18) which comprises
   (i) fusing a splenic cell from an animal such as a mouse which is immunized with a member selected from the group consisting of (a) a protein of SEQ ID NO: 1 or SEQ ID NO: 2 in the Sequence Listing or a salt thereof and (b) a peptide fragment thereof or a salt thereof, with an immortalized cell including an animal myeloma cell such as a mouse myeloma cell, and
   (ii) selecting an immortalized cell capable of producing a monoclonal antibody (MAb) to at least one member selected from the group consisting of (a) a protein of SEQ ID NO: 1 or SEQ ID NO: 2 in the Sequence Listing or a salt thereof and (b) a peptide fragment thereof or a salt thereof from the resultant hybrid cells.

In still another aspect, the present invention provides:
(20) a quantitative assay of trypsin and/or trypsin-like immunoreactivity in a sample to be measured which comprises
   reacting the antibody according to any of the above (1) to (5) with the sample together with a member selected from the group consisting of labeled canine trypsin or labeled canine trypsin-related substances, and
   measuring a ratio of labeled canine trypsin or labeled canine trypsin-related substances which are bound to the antibody;
(21) a quantitative assay of trypsin and/or trypsin-like immunoreactivity in a sample to be measured which comprises
   reacting an antibody insolubilized on a solid phase selected from the antibody according to any of the above (1) to (5) and a labeled antibody selected from the antibody according to any of the above (1) to (5) with the sample, and
   measuring either a label insolubilized on the solid phase or a label free of binding to the solid phase for insolubilization; and
(22) an assay system (for example, analytical test device)
   (a) being arranged such that a liquid sample (or moist sample) can be applied to a dry carrier,
   (b) comprising not only
      (i) a labeled specific binding reagent for a test target which labeled specific binding reagent is freely mobile within said carrier when in the moist state but also
      (ii) an unlabeled specific binding reagent for the test target which unlabeled specific binding reagent is permanently immobilized in a detection zone on a carrier material and is therefore not mobile in the moist state,
   (c) wherein the labeled reagent (i) is spatially distinct from the detection zone, and the relative positioning of the labeled reagent (i) and the detection zone is determined such that the liquid sample (or moist sample) applied to the system can pick up the labeled reagent (i) and thereafter permeate into the detection zone,
   the system incorporating means enabling the extent (if any) to which the labeled reagent (i) becomes bound in the detection zone to be observed, which comprises the following characteristics:
   the test target is selected from the group consisting of trypsin and trypsin-like immunoreactivity and
   at least one of the reagents is selected from the antibodies according to any of the above (1) to (5).

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

The term "and/or" used herein means the presence of both (i) a jointly connecting relation and (ii) a selectively connecting relation. For example, in the case of "trypsin and/or trypsin-like immunoreactivity", it is used in such a sense that said expression covers both (i) "trypsin and trypsin-like immunoreactivity" and (ii) "trypsin or trypsin-like immunoreactivity". In other cases, the term "and/or" is used in the same sense that it covers both (i) a jointly connecting relation and (ii) a selectively connecting relation as well.

### BRIEF DESCRIPTION OF THE DRAWINGS

FIG. 1 depicts Western blot immunostaining with anti-trypsin MAb. Purified trypsin (2.5µ g/lane) has been separated by SDS-PAGE (under a reducing condition), blotted onto nitrocellulose membrane and immunostained with anti-trypsin MAb 005-208.

FIG. 2 shows the relationship between the canine trypsin concentration in samples and A₄₀₅, obtained by 1 step sandwich EIA.

FIG. 3 shows the relationship between the canine trypsin concentration in samples and A₄₀₅, obtained by 2 step sandwich EIA.

### BEST MODES OF CARRYING OUT THE INVENTION

The present invention provides MAb to canine trypsin or canine trypsin-related substances, reagents comprising the said MAb, and assays using the said MAb for detecting and/or measuring canine trypsin and/or canine trypsin-like immunoreactivity. In an embodiment, the present invention provides MAb to at least one member selected from the group consisting of canine cationic trypsinogen, canine anionic trypsinogen, canine cationic trypsin, canine anionic trypsin and canine trypsin-related substances derived therefrom, immunoassays using the said MAb, and immunoassay reagents comprising the said MAb.

The present invention provides MAb to canine cationic trypsin or canine cationic trypsin-related substances, reagents comprising the said MAb, and assays using the said MAb for selectively detecting and/or measuring canine cationic trypsin and/or canine trypsin-like immunoreactivity. Accordingly, the present invention provides more accurate and meaningful assays and diagnoses enabling us to assay separately individuals of various canine trypsin and canine trypsin-like immunoreactivity species.

The term "canine trypsin or canine trypsin-related substance(s)" as used herein covers native and recombinant canine trypsin or canine trypsinogen, fragments obtained by fragmenting canine trypsin and canine trypsinogen, synthetic peptide fragments manufactured by selecting characteristic sequence areas based on amino acid sequences deduced from cloned and sequenced cDNA, followed by design and chemical synthesis of the polypeptides, proteins of SEQ ID NO:1 or SEQ ID NO:2 or a salt thereof, and peptide fragments or a salt thereof from the proteins of SEQ ID NO:1 or SEQ ID NO:2.

Canine trypsin exists in two subtypes, i.e., a cationic form (cationic) and an anionic form (anionic). Canine trypsinogen is processed to form active canine trypsin. Further, active canine trypsin also exists in vivo (for example, in blood) in complex forms wherein trypsin is coupled with inhibitors such as *α* 1-antitrypsin and *α* 2-macroglobulin. Active trypsin is degraded with peptidases, etc. and metabolized in vivo. Products obtained by transcription and translation from canine trypsin genes contain each a transport signal sequence though the signal sequence is removed in the process of their secretion. It is perceptible that any characteristic sequence region (peptide fragment) within the sequences of SEQ ID NOs: 1 and 2 in the Sequence Listing will be identified with a substance related to trypsin. Substances exerting trypsin-like immunoreactivity in vivo (for example, in blood, etc.) including, for example, inactive trypsinogen, active trypsin, complexes wherein trypsin is coupled in blood with inhibitors such as α 1-antitrypsin and α 2-macroglobulin, etc. are generally called "trypsin-like immunoreactivity" and the dynamics thereof has been drawing attention to diagnosis of a variety of diseases including, for example, acute pancreatitis, chronic pancreatitis, pancreatic cancer, renal insufficiency, further the end stage of chronic pancreatitis, exocrine pancreatic insufficiency, etc. and/or determination of the severity thereof.

Monoclonal antibodies related to the present invention can be produced via immunizing an animal with, as an immunogen, canine trypsin or a fragment thereof by known methods, said canine trypsin being purified from dog pancreas or dog pancreatic juice according to known techniques, followed by techniques known or widely applicable in the art, including the hybridoma method described in Kohler & Milstein, Nature, 256: 495-97, 1975 or according to modifications thereof. In these methods, any may be used as long as the immunogen is selected from the group consisting of native trypsin (or naturally-occurring trypsin), native trypsinogen, recombinant trypsin, synthetic peptides which have each an amino acid sequence selected from suitable areas present in the already reported amino acid sequences of trypsin or fragments derived from trypsin proteins, including, for example, synthetic peptides having each at least continuous 8 amino acid residues which are part of the amino acid sequence of trypsin, synthetic peptides having each an amino acid sequence with 14 amino acid residues, preferably peptides having each a sequence characteristic of trypsin, etc. Such synthetic peptides may be suitably designed by reference to the amino acid sequence of SEQ ID NO: 1 or SEQ ID NO: 2 as represented by the Sequence Listing (Mol. Cell. Biol., 5, pp.2669 to 2676, 1985).

The immunogen as used herein may be preferably canine trypsin (i.e., cationic trypsin or anionic trypsin) isolated and purified from dog pancreas, for example, according to the David et al. method (Biochem. Soc. Trans., Vol.11, No.4, 603rd MEETING, LIVERPOOL, PP.351, 1983). In particular, specific monoclonal antibodies are preferably produced when canine cationic trypsin thus purified is employed. For achieving objectives of acquiring antibodies which recognize a specific antigen determinant site of trypsinogen or trypsin,
(i) different amino acid sequence areas between cationic trypsin and anionic trypsin can be selected, then antigen peptides designed, synthesized, and subjected to immunization to produce antibodies enabling the selective assay of cationic trypsin or anionic trypsin;
(ii) homologous sequence areas or highly homologous sequence areas between cationic trypsin and anionic trypsin can be selected, then antigen peptides designed, synthesized, and subjected to immunization to produce antibodies enabling the assay of both cationic trypsin and anionic trypsin;
(iii) characteristic sequence areas can be selected which each contain a sequence area comprised of N-terminal residues (for example, N-terminal 7 to 8 residues of trypsinogen) existing in trypsinogen but removed in the processing of converting it into active trypsin or part or all of such a sequence area, then antigen peptides designed, synthesized, and subjected to immunization to produce antibodies enabling the selective assay of trypsinogen or trypsin; and further
(iv) sequence areas capable of binding to trypsin inhibitors or neighborhoods thereof can be selected, then antigen peptides designed, synthesized, and subjected to immunization to produce antibodies enabling the selective assay of trypsin or a trypsin-inhibitor complex (as required, with production and use of antibodies immunoreactive to said trypsin inhibitors); etc.

Trypsin is obtainable from in vivo and in vitro trypsin-producing cells including culture cells, removed tissues, culture tissues, etc. Examples of such trypsin sources are cells such as pancreatic acinous cells, pancreatic tissues, pancreatic juice, etc. Further, trypsin can be obtained in a recombinant form. For example, recombinant trypsin may be produced by utilization of, for example, trypsin-producing cells/tissues such as pancreatic acinous cells and pancreatic tissues according to recombinant DNA techniques including for example the method described in Mol. Cell. Biol., 5, pp.2669 to 2676, 1985. The trypsin products prepared according to the present invention and derivatives thereof are suitably employed as immunizing antigens. The trypsin products can be purified from various sources including for example antigen-producing materials such as culture cells, culture tissues and transformed cells by suitable combinations of widely known techniques per se for separation, isolation and purification. Such widely known techniques per se are, for example, salting out such as sedimentation with ammonium sulfate; gel filtration with Sephadex; ion exchange chromatography using carriers having, for example, a diethylaminoethyl or carboxymethyl group, etc.; hydrophobic chromatography using carriers having, for example, a hydrophobic group such as butyl, octyl, or phenyl, etc.; pigment (or chromophore) gel chromatography; electrophoresis; dialysis; ultrafiltration; affinity chromatography; high performance liquid chromatography; etc. Preferably, the target products can be isolated, separated and purified by polyacrylamide gel electrophoresis, affinity chromatography in which an antibody capable of specifically reacting with an antigen, such as a monoclonal antibody, is immobilized. Purified recombinant trypsin can be suitably employed as an immunizing antigen for producing monoclonal antibodies. Examples of such antigens are those purified by gelatin-agarose affinity chromatography, heparin-agarose chromatography, etc. Based on the acquired information on trypsin genes according to the present invention, peptides can be synthesized and then suitably applied as immunizing antigens to the production of monoclonal antibodies. Such peptides and derivatives thereof may be produced by conventionally known techniques for the synthesis of peptides. The production techniques may be any of solid phase methods and liquid phase methods. Examples of such methods include those described in, for example, E. Gross & J. Meienhofer (Ed.), "The Peptides: Analysis, Synthesis, Biology", Vols. 1 to 5, Academic Press, New York, U.S.A.; S. Udenfriend & J. Meienhofer (Ed.), "The Peptides: Analysis, Synthesis, Biology", Vols. 6 to 9, Academic Press, New York, U.S.A.; E. Atherton & R. C. Sheppard, "Solid Phase Peptide Synthesis, a Practical Approach", IRL Press, Oxford (1989); Nobuo IZUMIYA et al., "Peptide Synthesis", Maruzen Publishing Co., Ltd., Japan (1975); Nobuo IZUMIYA et al., "Fundamentals and Experiments of Peptide Synthesis", Maruzen Publishing Co., Ltd., Japan (1985); Haruaki YAJIMA (Ed.), Yoshio OKADA et al., "Pharmaceutical Research and Development, Second Edition, Vol. 14, Peptide Synthesis", Hirokawa Publishing Co., Ltd., Japan (1991), etc., the disclosures of which are hereby incorporated by reference.

Described herein below is the production of antibodies. It goes without saying that the monoclonal antibody according to the present invention may be a monoclonal antibody (MAb) obtained by utilizing cell fusion techniques with myeloma cells. The monoclonal antibodies of the present invention can be produced, for example, by the following processes:
1. Preparation of immunogenic antigens (immunogens)
2. Immunization of animals with immunogenic antigens
3. Preparation of myeloma cells
4. Cell fusion between antibody-producing cells and myeloma cells
5. Selection and cloning of hybridomas (hybrid cells)
6. Production of monoclonal antibodies

### 1. Preparation of immunogenic antigens

The antigen used includes recombinant canine trypsin or trypsinogen as prepared according to the aforementioned methods and suitable synthetic oligopeptides, chemically synthesized, based on information on sequenced canine trypsin or trypsinogen therein. A preferable example of the antigen is canine trypsin as isolated and purified from dog pancreas according to the David et al. report (Biochem. Soc. Trans., Vol.11, No.4, 603rd MEETING, LIVERPOOL, PP.351, 1983), including canine cationic trypsin and anionic trypsin.
A particularly preferred antigen is canine cationic trypsin thus purified. Further, the trypsin antigen may include the precursor of trypsin, trypsinogen, and an active form trypsin. For example, the antigen for such an immunogen may be a fragment derived from trypsin or a synthetic polypeptide fragment obtained via selecting characteristic sequence areas based on amino acid sequences deduced from the cloned and sequenced cDNA followed by design and chemical synthesis. Although such antigen proteins and polypeptides can be used to immunize animals after being mixed with suitable adjuvants without any modifications, they may also be used after formation of immunogenic conjugates, if necessary, followed by admixture with suitable adjuvants. The antigen fragments such as polypeptides may be coupled with various carrier proteins with aid of suitable coupling agents to form immunogenic conjugates such as hapten-proteins. The immunogenic conjugates can be used to design monoclonal antibodies that can react with (or recognize) specific sequences exclusively. A cysteine residue or the like can be added to the polypeptide thus designed so as to prepare an immunogenic conjugate easily. To couple with a carrier protein or the like, the carrier protein is first activated. This activation may include incorporation of an activated binding group thereinto, etc.

The activated binding groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, bacterial components such as BCG or the like.

### 2. Immunization of animals with immunogenic antigens

Animals can be immunized according to techniques as described in Shigeru MURAMATSU et al. ed., "Jikken Seibutsu Gaku Kouza 14, Men-eki Seibutsu Gaku (Lectures on Experimental Biology 14, Immunobiology)", Maruzen K.K., 1985; Nippon Seikagaku Kai (Biochemical Society of Japan) ed., "Zoku-Seikagaku Jikken Kouza 5, Men-eki Seikagaku Kenkyuho (Lectures on Biochemical Experiments (Second Series; 5), Methods for Immunological and Biochemical Study)", Tokyo Kagaku Dojin, Japan (1986); Nippon Seikagaku Kai (Biochemical Society of Japan) ed., "Shin-Seikagaku Jikken Kouza 12, Bunshi Men-eki Gaku III (Kougen-Koutai-Hotai) (New Lectures on Biochemical Experiments 12, Molecular Immunology III (Antigen-Antibody-Complement))", Tokyo Kagaku Dojin, Japan (1992); etc., the disclosures of which are hereby incorporated by reference. The adjuvant to be used with the antigen includes Freund's complete adjuvant, Ribi adjuvant, pertussis vaccine, BCG, lipid A, liposome, aluminium hydroxide, silica, etc. Immunization is carried out with animals, including mice such as BALB/c. The antigen dose is, for example, approximately 1 to 400 µ g/animal for mice. Generally, the antigen is injected intraperitoneally or subcutaneously into a host animal, followed by additional immunization by repeated courses wherein intraperitoneal, subcutaneous, intravenous or intramuscular administrations are carried out approximately 2 to 10 times at 1- to 4-week intervals, preferably 1- to 2-week intervals. For immunization, BALB/c mice, as well as F1 mice between BALB/c mice and other mice, etc. can be used. As required, the degree of animal immunization can be assessed by constructing a an antibody titre assay system and measuring the titre of an antibody.

### 3. Preparation of myeloma cells

Immortal cell strains (tumor cell lines) to be used for cell fusion can be selected from non-immunoglobulin-producing cell lines. The cell strains to be used for cell fusion may include, for example, P3-NS-1-Ag4-1 (NS-1, Eur. J. Immunol., 6: 511 to 519, 1976), SP-2/0-Ag14 (SP-2, Nature, 276: 269 to 270, 1978), mouse myeloma MOPC-21 cell line-derived P3-X63-Ag8-U1 (P3U1, Curr. topics in Microbiol. Immunol., 81: 1 to 7, 1978), P3-X63-Ag8 (X63, Nature, 256: 495 to 497, 1975), P3-X63-Ag8-653 (653, J. Immunol., 123: 1548 to 1550, 1979), etc. 8-Azaguanine resistant mouse myeloma cell lines can be subcultured in a medium for cell culture, such as Dulbecco's modified Eagle's medium (DMEM), Iscove's modified Dulbecco's medium (IMDM) and RPMI-1640 medium, supplemented with antibiotics such as penicillin, streptomycin, amikacin or the like, fatal calf serum (FCS) or the like and 8-azaguanine (for example, 5 to 45 µg/ml). The specified number of cell lines can be prepared by passage in the normal medium 2 or 5 days before cell fusion. The cell lines to be used may be cultured on the normal medium after the frozen and preserved strains have been completely thawed at approximately 37°C and have been washed 3 or more times with the normal medium such as RPMI-1640, and the specified number of cell strains may be prepared.

### 4. Cell fusion between antibody-producing cells and myeloma cells

After animals such as mice are immunized according to the above step 2, their spleens are removed on 2 to 5 days from final immunization, and the spleen cell suspension is obtained. In addition to the spleen cells, lymph node cells at various sites of organisms can be obtained and used for cell fusion. The spleen cell suspension thus obtained and the myeloma cell strains obtained by the above step 3 are placed in a medium such as minimum essential medium (MEM), DMEM or RPMI-1640 medium, followed by addition of an agent for cell fusion, such as polyethylene glycol. The agent for cell fusion may also include those known in the art, including inactivated HVJ (Hemagglutinating virus of Japan, Sendai virus) in addition to the above agent. Preferably, 30 to 60% polyethylene glycol can be added at a dose range of 0.5 to 2 ml. Polyethylene glycol with 1,000 to 8,000 in molecular weight can be employed, more preferably, polyethylene glycol between 1,000 and 4,000 in molecular weight. The preferred concentration of polyethylene glycol in the fusion medium is between 30 and 60%. As required, a small amount of dimethyl sulfoxide or the like is added to promote fusion. The ratio of spleen cells (lymphocytes) : myeloma cell lines to be used for fusion is preferably 1:1 to 20:1, and preferably falls between 4:1 and 10:1. The fusion reaction is conducted for 1 to 10 minutes, before the addition of a medium such as RPMI-1640 medium. Fusion reaction can be done several times. After fusion reaction, cells are separated by a centrifuge, then transferred to the selection medium.

### 5. Selection and cloning of hybridomas (hybrid cells)

The selection media include conventionally known "HAT medium", i.e., FCS-containing MEM, DMEM, IMDM, RPMI-1640 medium, etc., supplemented with hypoxanthine, aminopterin, and thymidine. The replacement for the selection medium is to replenish an amount equivalent to the capacity dispensed to the medium plate on the following day, after which the medium is replaced by half an amount in HAT medium every 1 to 3 days. The replacement can be modified depending on situations. Eight to sixteen days after fusion, the medium may be replaced every 1 to 4 days with conventionally known "HT medium" wherein aminopterin is excluded from HAT medium. As a feeder cell, for example, mouse thymocyte can be used, which is sometimes effective.

The supernatant of the culture well with highly growing hybridoma is screened by using trypsin or a peptide fragment thereof as an antigen or by using a labeled anti-mouse antibody for measuring target antibodies, with a measuring system such as radioimmunoassay (RIA), enzyme-linked immunosorbent assay (ELISA), fluorescence immunoassay (FIA) or by the fluorescence activated cell sorter (FACS), etc. The target antibody-producing hybridoma is cloned. Cloning is carried out by picking up colonies in the agar medium or by the limiting dilution. The limiting dilution is preferred. Cloning should be performed several times.

### 6. Production of monoclonal antibodies

The obtained hybridoma cells are cultured in a suitable growth medium such as FCS-containing or FCS-free MEM, DMEM, IMDM, RPMI-1640 medium or the like, and a desired monoclonal antibody can be obtained from the culture supernatant. Large amounts of monoclonal antibodies can be produced by propagating hybridomas as ascites tumors, etc. In this case, each hybridoma is implanted intraperitoneally in a histocompatible animal isogenic to an animal from which the myeloma cell is derived and is propagated. Or each hybridoma can be inoculated, for example, in nude mice, and propagated to produce the monoclonal antibody in the ascites of the animals. The produced monoclonal antibody can be collected from the ascetic fluid and obtained. Prior to implantation of hybridomas, the animal is pre-treated intraperitoneally with mineral oils such as pristane (2,6,10,14-tetramethylpentadecane). After the pretreatment, the hybridoma can be propagated therein and the ascitic fluid can be harvested. The ascitic fluid can be used as a monoclonal antibody without purification or after purification by conventionally known methods, including salting out such as precipitation with ammonium sulfate, gel filtration with Sephadex, ion exchange chromatography, electrophoresis, dialysis, ultrafiltration, affinity chromatography, high-performance liquid chromatography, etc. The isolated or purified products can be employed as monoclonal antibodies. Preferably, the monoclonal antibody-containing ascitic fluid is fractionated with ammonium sulfate and separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize); affinity chromatography with immobilized protein A; etc.

It is possible to produce antibodies by recombinant DNA techniques wherein the antibody thus obtained in a large amount is sequenced and/or a nucleotide sequence coding for the antibody obtained from the hybridoma cell line is employed.

These antibodies may be treated with enzymes such as trypsin, papain, pepsin or the like and occasionally be subjected to reduction to produce antibody fragments including Fab, Fab', and F(ab')₂. These antibody fragments may be occasionally used.

The antibody to be labeled with a marker may include IgG fractions, and specific bonding fragments Fab' obtainable by reduction after pepsin digestion. The labels include enzymes (peroxidase, alkaline phosphatase, or β-D-galactosidase or the like), chemical substances, fluorescences, radioisotopes, or the like, as disclosed hereinbelow.

In the present invention, detection and measurement can be carried out by immunoagglutination. For example, antibodies carried on latex particles can immunoreact. The detection and measurement can be carried out by immunostaining including, for example, staining of tissues and cells, immunoassays including, for example, competitive immunoassay and non-competitive immunoassay, radioimmunoassay, ELISA, or the like. The detection and measurement can also be carried with or without B-F separation. Preferably, the detection and measurement is carried out by means of radioimmunoassay, enzyme immunoassay or sandwich assay. In the sandwich-type assay, one of the antibodies against trypsin is detectably labeled.
The other antibody capable of recognizing the same antigen is immobilized on a solid phase.

Incubation is carried out to sequentially react a sample to be assayed, labeled antibodies, and immobilized antibodies as required. After the non-binding antibodies are separated, the label is detected or measured. The amount of the measured label is proportional to the amount of antigen, i.e., trypsin. For this assay, simultaneous sandwich assay, forward sandwich assay, or reverse-sandwich assay or the like is called, based on the difference according to the addition sequence of the insolubilized antibody and the labeled antibody. For example, washing, stirring, shaking, filtration, pre-extraction for antigen, etc. is optionally adopted in the measurement process under specific conditions. The other measurement conditions such as specific regents, concentration of buffers, temperature or incubation time can vary according to the elements, such as concentration of the antigens in the sample or the nature of samples to be measured. Any person ordinary skilled in the art can suitably select and determine optimal conditions effective for each measurement while using the general experimentation and perform the selected measurement.

Various carriers on which antigens or antibodies can be immobilized (or insolubilized) are available in the art, and they can be arbitrarily and suitably selected in the present invention. For the immobilization, various carriers which can be used for antigen-antibody interactions are known. It goes without saying that any well-known carrier can be selected and used in the present invention. Preferred examples are inorganic materials including, for example, glass such as activated glass and porous glass, silica gel, silica-alumina, alumina, magnetized iron, magnetized alloy, etc.; organic polymers including, for example, polyethylene, polypropylene, polyvinyl chloride, polyvinylidene fluoride, polyvinyl acetate, polymethacrylate, polystyrene, styrene/butadiene copolymer, polyacrylamide, cross-linked polyacrylamide, styrene/methacrylate copolymer, polyglycidyl methacrylate, acrolein/ethylene glycol dimethacrylate copolymer, etc., cross-linked albumin, collagen, gelatin, dextran, agarose, cross-linked agarose, natural or modified cellulose such as cellulose, microcrystalline cellulose, carboxymethylcellulose, cellulose acetate and the like, cross-linked dextran, polyamide such as nylon, polyurethane, polyepoxy resin and the like; those obtained by emulsifying polymerization thereof; cells, erythrocytes and the like; and those into which a functional group may be introduced, as required, by using a silane coupling agent.

Also included are solid materials such as filter paper, beads, inner wall of test container such as test tube, titer plates, titer wells, glass cells, cells made of synthetic materials such as plastic resin cells, glass rods, rods made of synthetic materials, rods thickened or thinned at the end, rods whose end is round or flat, thin-plated rods, and surfaces thereof.

Antibodies can be coupled with these carriers. Preferably the monoclonal antibodies according to the present invention can be coupled to the carriers, said antibodies being capable of specifically binding with trypsin. Coupling between the carrier and those associated with these antigen-antibody interactions can be carried out by techniques including physical method such as adsorption; a chemical method using a coupling agent, etc. or an activated reactant; a method using a chemically interactional coupling.

The label may include enzymes, enzyme substrates, enzyme inhibitors, prosthetic groups, coenzymes, enzyme precursors, apoenzymes, fluorescent substances, pigments, chemical luminescent compounds, light-emitting substances, coloring substances, magnetic substances, metal particles such as gold colloids, colloidal particles, microparticles, radioactive substances, biotin, and the like.

The enzyme may include dehydrogenases, oxidoreductases such as reductases and oxidases; transferases that catalyze the transfer of an amino, carboxyl, methyl, acyl, phosphate group or the like; hydrolases that hydrolyze an ester, glycoside, ether, peptide bond or the like; lyases; isomerases; ligases; and the like. Plural enzymes can be used in a conjugated form for detection (for example, enzymatic cycling may also be utilizable).

The colloidal metal particle labels may be preferably those capable of providing a visualizable signal and include particles which are comprised of species selected from metals, metal oxides, metal hydroxides, metal salts, etc. Particles may comprise pure metal, metal compounds or mixtures thereof. Further, the particles may be those comprising cores formed from polymers such as synthetic resins which are coated with metal materials such as metals or metal compounds. Examples of suitable metals and metal compounds include metals such as gold, silver, platinum and copper; metal compounds such as silver hydroxide, silver bromide, silver iodide, iron oxide, iron hydroxide, aluminum hydroxide, aluminum oxide, chromium hydroxide, copper sulfate, mercury sulfate and titanium dioxide; and the like. Preferred colloidal metal particle labels include gold colloids (gold sols), silver colloids, iron oxide colloids, etc. The colloidal particles for labels may also include non-metal particles formed from selenium, tellurium, sulfur, etc. Further, the colloidal particle label may include sol particles of dyes. The particles must be detectable and preferably produce a visually detectable signal when present in relatively low concentrations.

Colloidal particles such as colloidal metal particles may be produced according to methods generally known in the art. For example, methods for the production of gold sol (colloidal gold) particles are disclosed in Nature, 241, 20, (1973), etc., the disclosure of which is hereby incorporated by reference. Gold particles may be produced by methods wherein a solution of gold chloride is heated to boiling and is then mixed with a solution of sodium citrate, etc. to reduce the gold chloride, etc. Soon after mixing of the two solutions the boiling solution turns a faint blue indicating the onset of nucleation soon thereafter the blue color changes to red indicating the formation of mono-disperse particles. The resulting particle sizes may be controlled by variation of the concentration of the sodium citrate solution. The colors of the visually detectable signal from the metal particle label is dependent upon the identity and particle size of the metal particle. For example, colloidal gold particles produce colors varying from orange to reddish violet depending upon the particle size of the sol. The particle size of the colloid particle can be suitably selected to achieve most optimal detection/assay sensitivity.

Typical radioactive isotopes for the label include [³²p], [¹²⁵I], [¹³¹I], [³H], [¹⁴C], [³⁵S], and the like.

Typical enzymes for the label include peroxidases such as horseradish peroxidase (HRP); galactosidase such as E. coli β-D-galactosidase; maleate dehydrogenase; glucose-6-phosphate dehydrogenase; glucose oxidase; glucoamylase; acetylcholine esterase; catalase; alkaline phosphatase such as calf intestinal alkaline phosphatase and E. coli alkaline phosphatase, and the like.

In the case where alkaline phosphatase is used, fluorescence or emitted light can be measured by using a substrate such as umbelliferone derivatives including 4-methylumbellipheryl phosphate; phenol phosphate derivatives including nitrophenyl phosphate; enzymatic cycling systems utilizing NADP; luciferin derivatives; dioxetane derivatives; and the like. It is also possible to use a luciferin/luciferase system. When catalase is used, the reaction takes place with hydrogen peroxide to produce oxygen which can be detected with an electrode or the like. The electrode may be a glass electrode, an ionic electrode using an insoluble or hardly soluble salt membrane, a liquid-membrane type electrode, a polymer membrane electrode and the like.

It is possible to replace the enzyme label with a biotin label and an enzyme-labeled avidin (streptoavidin). For the label, a plurality of many different kinds of labels or markers can be used. In this case, it is possible to perform plural measurements continuously or discontinuously and/or simultaneously or separately.

According to the present invention, a signal can be formed by using a combination of 4-hydroxyphenylacetic acid, 1,2-phenylenediamine, tetramethylbenzidine, or the like, with horseradish peroxidase, by using a combination of umbelliferyl galactoside, nitrophenyl galactoside, or the like, with enzyme reagents such as β-D-galactosidase and glucose-6-phosphoric acid dehydrogenase. There can be further used those that are capable of forming a quinol compound such as hydroquinone, hydroxybenzoquinone or hydroxyanthraquinone, a thiol compound such as lipoic acid or glutathione, phenol derivatives or ferrocene derivatives by utilizing the action of enzymes.

The fluorescent substance and chemiluminescent compounds may include fluorescein isothiocyanate, Rhodamine derivatives such as Rhodamine B isothiocyanate, and tetramethyl Rhodamine isothiocyanate, dansyl chloride (5-(dimethylamino)-1-naphtalenesulfonyl chloride), dansyl fluoride, fluorescamine (4-phenylspiro[furan-2(3H),1'-(3'H)-isobenzofuran]-3,3'-dione), phycobiliprotein, acridinium salts, luminol compounds such as lumiferin, luciferase, and aequorin, imidazole, oxalic acid ester, rare earth chelate compounds, cumarin derivatives, etc.

The labelling can be accomplished by utilizing the reaction of a thiol group with a maleimide group, the reaction of a pyridyldisulfide group with a thiol group, the reaction of an amino group with an aldehyde group, etc. Additionally, it can be selected from widely known methods, methods that can be easily put into practice by an artisan skilled in the art, or any of methods modified therefrom. The coupling agents used for producing the foregoing immunoconjugate or for coupling with carriers are also applicable and usable.

The coupling agents include, for example, glutaraldehyde, hexamethylene diisocyanate, hexamethylene diisothiocyanate, N,N'-polymethylene bisiodoacetamide, N,N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio)propionate (SPDP), N-succinimidyl 4-(N-maleimidometyl)cyclohexane-1-carboxylate (SMCC), N-sulfosuccinimidyl 4-(N-maleimidomethyl)-cyclohexane-1-carboxylate, N-succinimidyl (4-iodoacetyl)-aminobenzoate, N-succinimidyl 4-(1-maleimidophenyl)butyrate, N-(ε -maleimidocaproyloxy)succinimide (EMCS), iminothiolane, S-acetylmercaptosuccinic anhydride, methyl-3-(4'-dithiopyridyl)propionimidate, methyl-4-mercapto-butyrylimidate, methyl-3-mercaptopropionimidate, N-succinimidyl-S-acetylmercaptoacetate, etc.

According to the assay of the present invention, substances to be measured can be made to react sequentially with labeled antibody reagents such as monoclonal antibodies labeled with enzymes or the like, and with antibodies coupled on a carrier, or all the members can be reacted each other simultaneously. The order of adding reagents (members) may vary depending on the type of carrier system selected. In the case where beads such as sensitized plastics are used, the labeled antibody regents such as monoclonal antibodies labeled with enzymes or the like are first put in a suitable test tube, together with a sample including substances to be measured, followed by addition of the plastic beads. Measurement can be then carried out.

For quantitative measurements according to the present invention, the immunological measurement is applied. For the measurement, the solid phase carriers used may include various materials and shapes which can be selected from balls, microplates, sticks, microparticles, test tubes, and the like, made of polystyrene, polycarbonate, polypropylene, polyvinyl and other materials capable of well adsorbing proteins such as antibodies.

The measurement can be carried out in a suitable buffer system so as to maintain in optimal pH (for example, between about pH 4 and 9). In particular, the preferred buffers may include acetate buffer, citrate buffer, phosphate buffer, Tris buffer, triethanolamine buffer, borate buffer, glycine buffer, carbonate buffer, Tris-hydrochloride buffer, etc. The buffers can be used optionally in a mixed form at an arbitrary rate. Preferably, the antigen-antibody interaction is carried out at a temperature between about 0 and 60°C.

The enzyme-labeled antibody (for example, enzyme-labeled monoclonal antibody, etc.) regents, the carrier-coupled antibody (for example, carrier-coupled monoclonal antibody, etc.) regents, and samples to be measured can be incubated until equilibrium is reached. However, the reaction can be stopped after limited incubation by separating the solid phase from the liquid phase at a time well before the antigen-antibody interaction equilibrates, and the degree of the presence of labels such as enzymes in either of the liquid and solid phases can be measured. Measurement operation can be performed by using automated measuring instruments, and data can be measured by permitting a substrate to be converted by the action of enzymes and by detecting produced indication signals with a luminescence detector, a photo detector or the like.

The immunoassays can be constructed such that practices are not requiring special skill but also simply and readily applicable even in uses at home, clinic, doctor's office, etc., and assay results will be quickly obtainable in a reproducible manner. An example thereof includes an assay system (for example, analytical test device) which
(a) is arranged such that a liquid sample (or moist sample) can be applied to a dry carrier (for example, porous carrier, etc.), and
(b) comprises not only
   (i) a labeled specific binding reagent for a test target which labeled specific binding reagent is freely mobile within said carrier when in the moist state but also
   (ii) an unlabeled specific binding reagent for the test target which unlabeled specific binding reagent is permanently immobilized in a detection zone on a carrier material and is therefore not mobile in the moist state,
(c) wherein the labeled reagent (i) and the detection zone are spatially isolated each other, and the relative positioning of the labeled reagent (i) and the detection zone is determined such that the liquid sample (or moist sample) applied to the system can elute the labeled reagent (i) and thereafter permeate into the detection zone,
the system incorporating means enabling the extent (if any) to which the labeled reagent (i) becomes bound in the detection zone to be observed.

Preferred examples of said assay systems (for example, test devices) include those generally known as lateral flow devices. Examples thereof are those devices disclosed in Japanese Patent No.2,705,767 and constructs with modifications by reference to the patent document.

A typical embodiment of the present invention is a assay system wherein a dry carrier (e.g., porous carrier, etc.) carries a labeled reagent (i) in a first zone, an unlabeled reagent is immobilized in a detection zone which is spatially distinct from the first zone, and the two zones are arranged such that a liquid sample (or. moist sample) applied to the carrier (e.g., porous carrier, etc.) will permeate via the first zone into the detection zone. Thus, the assay system (for example, analytical test device) can be constructed such that the labeled reagent (i) is capable of participating with the unlabeled reagent (ii) in either a "sandwich" or "competitive" assay. In another aspect, the present invention also provides a method in which the assay system (e.g., test device) is used such that a liquid sample (such as an aqueous sample) suspected of containing a test target is applied to a carrier, the sample permeates by capillary action, etc. through the carrier (e.g., porous carrier, etc.) via the first zone into the second zone and the labeled reagent (i) migrates therewith from the first zone to the second zone, the presence of the test target in the sample being determined by observing test results including the extent (if any) to which the labeled reagent (i) becomes bound in the second zone.

For example, when MAb to canine trypsin is used for the labeled reagent (i) and the unlabeled reagent (ii), respectively, the labeled reagent (i), the antigen to be detected (if present in the sample) and the immobilized unlabeled reagent cooperate together in a "sandwich" reaction. This results in the labeled reagent being bound in the second zone if antigen to be detected is present in the sample. In another embodiment of the present invention, the labeled reagent is either a labeled antigen itself such as canine trypsin which has been conjugated with a label, or a target antigen analogue which similarly has been conjugated with a label. In this instance, the labeled antigen (such as labeled canine trypsin) or antigen analogue will migrate through the carrier (for example, a porous carrier, etc.) into the second zone and bind with the immobilized reagent. Any target antigen present in the sample will compete with the labeled reagent in this binding reaction. Such competition will result in a reduction in the amount of labeled reagent binding in the second zone, and a consequent decrease in the intensity of the signal observed in the second zone in comparison with the signal that is observed in the absence of antigen in the sample.

In a preferred embodiment, the assay system can be constructed such that a liquid sample (or moist sample) can be applied to a carrier (e.g., porous carrier, etc.) via a bibulous sample receiving member which has a function of receiving the liquid sample (or moist sample) and giving the sample to the carrier (e.g., porous carrier, etc.).
In this case, the bibulous sample receiving member may contain the labeled reagent (i) in a dry state. The receiving member includes preferably those materials capable of absorbing liquid rapidly. As long as it has such properties, it can be made from any bibulous, porous or fibrous material. The porosity of the material can be mostly unidirectional or multidirectional (or omnidirectional). The unidirectional material may refer to substances with pores or fibers running wholly or predominantly parallel to an axis of the member. When the material has multidirectional porosity, etc., the member will have an amorphous sponge-like structure. The materials as used herein include porous plastic materials. Examples of the material are those comprised of polypropylene, polyethylene, polyvinylidene fluoride, ethylene vinyl acetate, acrylonitrile, polytetrafluoroethylene, etc. It can be advantageous to pre-treat the member with a surface-active agent during manufacture, as this can reduce any inherent hydrophobicity in the member and therefore enhance its ability to absorb and deliver a moist sample rapidly and efficiently. The receiving members can also be made from paper or other cellulosic materials such as nitrocellulose.

In these assay systems, the label can be any entity the presence of which can be readily detected. Preferably the label is a direct label, i.e., an entity which, in its natural state, is readily visible either to the naked eye, or with the aid of an optical filter and/or applied stimulation, e.g., UV light, etc. to promote fluorescence, etc. The label includes, for example, microparticles including colloidal metal particles such as gold colloids (gold sols), colloidal dye particles, colored latex particles, etc. A porous carrier material is usually used for the carrier. Preferably the material is, for example, nitrocellulose, etc. It can be selected from natural and synthetic polymers and derivatives thereof, including a filter paper. Examples of the polymers and derivatives include those listed for the aforementioned carrier. Preferably the carrier material as used herein is in the form of a strip or sheet. When the carrier is formed in a strip or sheet shape, the reagents are preferably painted on spacially distinct zones, and the liquid sample (or moist sample) is allowed to permeate through the sheet or strip from one side or end to another. Further, the carrier can be optionally backed with a transparent moisture-impermeable material layer such as a plastic sheet.

The assay system can be designed to have a "control zone". The control zone can be constructed to convey a signal to the user that the assay system (e.g., test device) has acted or worked, and/or the test has normally been achieved. For example, the control zone can be loaded with a binding reagent such as an antibody that will bind to the labeled antibody from the first zone, to confirm that the sample has permeated the test carrier. For instance, if the labeled antibody is one monoclonal antibody that has been derived using a murine hybridoma, the control zone can be loaded with an "anti-mouse antibody". Alternatively, the control zone can optionally contain a suitable reagent or means such as a reagent that, when moistened, produces a color change or color formation.

The reagents can be painted on or retained in the carrier material in a variety of ways. Liquid reagents are applied to carrier materials by various techniques including, for example, painting or printing on, or impregnation into the carrier material with a micro-syringe, micro-pipette, pen equipped with a regulator, ink-jet printing device, etc.

In the antigen-antibody interaction, adequate means can be taken so as to stabilize regents to be used, samples to be assayed, and labels such as enzymes, respectively, and/or to stabilize antigen-antibody interactions per se. Further, for eliminating non-specific reaction, reducing inhibitory influences acting thereon, and/or activating assay reaction, proteins, stabilizers, surfactants, chelating agents or the like can be added to solutions which are incubated. The chelating agent is more preferably ethylenediamine tetraacetate (EDTA).

The blocking techniques for preventing non-specific binding reaction may be employed, which techniques are generally employed in the art or well-known among the persons skilled in the art. The blocking can be achieved by treatments with normal serum proteins, albumin, skim milk or the like from mammals, etc., fermented milk products, collagen, gelatin, or the like. These methods or techniques can be used without any limitation since the purpose is to prevent non-specific binding reaction.

The samples to be assayed according to the present invention may include various types of solutions such as colloid solutions, non-fluid samples and the like. Preferably, the samples are biological samples including, for example, blood, serum, plasma, articular fluid, saliva, urine, stools, pancreas, pancreatic juice, any other body fluids, cell culture medium, tissue culture medium, tissue homogenate, biopsy samples, tissues, cells and the like.

In applying each of those immunometric assay (immunoassays) to the assays of the present invention, it is not necessary to set up any special condition, operation, etc. therefor. An assay system of the present invention for native canine trypsin or dog-derived proteins having a substantially equivalent activity thereto may be constructed taking the technical knowledge owned by the persons skilled in the art plus the conventional conditions and operations for each of the methods into consideration. With details of those conventional technical means, a variety of reviews, reference books, etc. may be referred to. They are, for example, Hiroshi Irie (ed), "Radioimmunoassay", Kodansha, Japan, 1974; Hiroshi Irie (ed), "Radioimmunoassay; Second Series", Kodansha, Japan, 1979; Eiji Ishikawa et al. (ed), "Enzyme Immunoassay", Igaku Shoin, Japan, 1978; Eiji Ishikawa et al. (ed), "Enzyme Immunoassay" (Second Edition), Igaku Shoin, Japan, 1982; Eiji Ishikawa et al. (ed), "Enzyme Immunoassay" (Third Edition), Igaku Shoin, Japan, 1987; "Methods in Enzymology", Vol. 70 (Immunochemical Techniques, Part A), Academic Press, USA; "Methods in Enzymology", Vol. 73 (Immunochemical Techniques, Part B), Academic Press, USA; "Methods in Enzymology", Vol. 74 (Immunochemical Techniques, Part C), Academic Press, USA; "Methods in Enzymology", Vol. 84 (Immunochemical Techniques, Part D: Selected Immunoassays), Academic Press, USA; "Methods in Enzymology", Vol. 92 (Immunochemical Techniques, Part E: Monoclonal Antibodies and General Immunoassay Methods), Academic Press, USA; "Methods in Enzymology", Vol. 121 (Immunochemical Techniques, Part I: Hybridoma Technology and Monoclonal Antibodies), Academic Press, USA; etc., the disclosures of which are hereby incorporated by reference.

In typical cases, the object of the present invention is also to provide excellent methods for selectively quantitating each individual free precursor and active trypsin in samples to be tested and reagent kits for such methods, said method and kit using a member selected from the group consisting of monoclonal antibodies to trypsin, monoclonal anti-trypsin antibodies on solid phase carriers, and, as required, inhibitors against trypsin. Embodiments of the present invention are construed to include all of each individual reagent in reagent kits enabling us to selectively assay such respective free precursors and active trypsin. Further, the objectives of the present invention are to provide methods and diagnostic drugs enabling us to selectively quantitate each individual free precursor and active trypsin by application of the aforementioned quantitative assay and to monitor pancreatic diseases, etc. and diagnostic drugs. Accordingly, embodiments of the present invention are construed to include not only a variety of utilization of the aforementioned reagents in medical and physiological fields but also all applications of the aforementioned reagents with the aim of studying, analyzing and assaying cells & tissues of animals such as dog, including pancreas.

Utilization of the aforementioned various embodiments of the present invention will lead to (a) means for examinations/measurements useful in studies related to the diagnosis of canine diseases or disorders (including pancreatic diseases such as acute pancreatitis, chronic pancreatitis, pancreatic cancer and exocrine pancreatic insufficiency; visceral diseases such as renal insufficiency; etc.) and dog's health and (b) a variety of techniques suitable for other medical and physiological applications.

As aforementioned, canine cationic trypsin is used as an immunogen to produce monoclonal antibodies to canine trypsin in accordance with the present invention. Thus, it is entirely novel that attention is paid to cationic molecule species regarding trypsin and MAb to such species is produced. Similarly, cationic trypsin may be used as an immunogen to produce monoclonal antibodies specifically immunoreactive to the antigen with respect to human, thereby achieving selective immunoassays of trypsin and/or trypsin-like immunoreactivity. Human cationic trypsin as used herein may be those samples isolated and purified from human pancreatic juice, for example, according to Biochemistry, Vol.8, No.7, pp.2884-2889, 1969.
It is contemplated that the subject matter of the present invention can be employed not only in the following examples which will be described in detail but can be modified (without any limitation) by one of skill in the art for carrying out numerous embodiments without departing from the inventive concepts embodied herein.

For terms (words) and/or abbreviations used in the specification and in the drawings, they must conform with an "IUPAC-IUB Commission on Biochemical Nomenclature" or are based on the meanings of the terms which are commonly used in the art.

The mouse-derived monoclonal anti-trypsin antibody-producing hybridoma, designated 004-203, obtained in Example 2(g) mentioned hereinbelow has been deposited as from July 28, 1998 (original deposit date) with the National Institute of Bioscience and Human Technology (NIBH), Agency of Industrial Science and Technology, Ministry of International Trade and Industry, Japan, located at 1-3, Higashi 1-chome, Tsukuba-shi, IBARAKI (Zip Code: 305-8566), JAPAN and has been assigned the Accession Number FERM P-16914. The original deposit of the hybridoma 004-203 has been transferred to one under the Budapest Treaty by a request dated July 30, 1999 and is on deposit with the Accession Number FERM BP-6808 under the terms of the Budapest Treaty at NIBH.

The mouse-derived monoclonal anti-trypsin antibody-producing hybridoma, designated 005-201, obtained in Example 2(g) mentioned hereinbelow has also been deposited as from July 28, 1998 with NIBH and has been-assigned the Accession Number FERM P-16915.

The mouse-derived monoclonal anti-trypsin antibody-producing hybridoma, designated 008-207 has been deposited as from March 9, 1999 (original deposit date) with NIBH and has been assigned the Accession Number FERM P-17290. The original deposit of the hybridoma 008-207 has been transferred to one under the Budapest Treaty by a request dated July 26, 1999 and is on deposit with the Accession Number FERM BP-6795 under the terms of the Budapest Treaty at NIBH. The mouse-derived monoclonal anti-trypsin antibody-producing hybridoma, designated 009-303 has been deposited as from March 9, 1999 (original deposit date) with NIBH and has been assigned the Accession Number FERM P-17291. The original deposit of the hybridoma 009-303 has been transferred to one under the Budapest Treaty by a request dated July 26, 1999 and is on deposit with the Accession Number FERM BP-6796 under the terms of the Budapest Treaty at NIBH.

### EXAMPLES

Described below are examples of the present invention which are provided only for illustrative purposes, and not to limit the scope of the present invention. It should be understood that numerous variations, equivalents and modifications that would be within the purview of one skilled in this art can be effected without departing from the true spirit and scope of the invention.

### Example 1

### Purification of Canine Trypsin

Canine trypsin was isolated and purified from dog pancreas by the method of David et al. (Biochem. Soc. Trans., Vol.11, No.4, 603rd MEETING, LIVERPOOL, PP.351, 1983) with the modifications according to methods for the purification of human trypsin, including, for example, the method described in Biochemistry, Vol.8, No.7, pp.2884-2889, 1969 and methods for the purification of bovine trypsin, including, for example, the method described in Sidney P. Colowick and Nathan O. Kaplan (Ed.), Methods in Enzymology, Vol.2 "Preparation and Assay of Enzymes", 1955, Academic Press (USA).

To a dog pancreas (27 g) was added 0.125 N sulfuric acid (150 mL), the mixture was homogenized with a blender and then incubated for 18 hours at 4°C. Next, the mixture was centrifuged (1500× g) to give a supernatant (1). To the resultant precipitate was added 0.125 N sulfuric acid (50 mL), the mixture was homogenized with a blender and then centrifuged (1500 × g) to give a supernatant (2).
The resulting supernatants (1) and (2) were combined to give an extract liquid (185 mL), to which was added ammonium sulfate (19.6 g) to bring the final ammonium sulfate concentration to 0.8 M. The mixture was incubated for 24 hours at 4°C and then centrifuged (23,000× g) to give a supernatant (a) separate from a precipitate. To the resultant precipitate was added 0.8 M ammonium sulfate (40 mL), the precipitate mixture was well dispersed and then centrifuged (23,000× g) to give a supernatant (b) separate from a precipitate. The resulting supernatants (a) and (b) were combined to give a liquid (220 mL), to which was added ammonium sulfate (64 g) to bring the final ammonium sulfate concentration to 3.0 M. The mixture was incubated for 24 hours at 4°C and then centrifuged (23,000× g) to give a precipitate separate from a supernatant. To the resultant precipitate was added 3.0 M ammonium sulfate (60 mL), the precipitate mixture was well dispersed and then centrifuged (23,000 × g) to give a precipitate separate from a supernatant.

The resultant precipitate was dissolved in distilled water (10 mL) and dialyzed against 1 mM hydrochloric acid. Next, the crude enzyme sample solution was lyophilized to give a dry product (0.4 g) which was stored at -80°C until next purification.

The freeze-dried crude enzyme sample (0.4 g) was dissolved in 10 mL of a salt solution (0.2 M NaCl/0.05 M CaCl₂, pH 2.6). The enzyme sample solution was purified by gel chromatography. The gel chromatography was carried out on a Sephadex G-75 (Pharmacia) column (3.8 cm²× 90 cm) under the following conditions:
mobile phase: 0.2 M NaCl/0.05 M CaCl₂, pH 2.6,
flow rate: 12 mL/hr, and
fraction size: 5 mL/fraction.
Each fraction was tested for absorbance at 280 nm (A₂₈₀) and enzyme activity. The enzyme assay was carried out as follows: To a small test tube was added 1.7 mL of 2 mM CaCl₂/0.05 M Tris-HCl, pH 8.0, and then 100 µL each of suitably diluted eluate samples. To the mixture was added 200 µL of 1 mM benzoyl arginine ethyl ether (BAEE) to initiate a reaction.
The time-course of reaction was observed by measuring the absorbance at 253 nm (A₂₅₃), provided that it was defined as 0.001/min.=1 BAEE Unit.

Potential active fractions Nos.36 to 42 were pooled together and subjected to dialysis against 1 mM hydrochloric acid. Next, after dialysis, the sample was lyophilized to give a dry product (0.2 g).

The freeze-dried sample (0.2 g) was dissolved in 5 mL of a buffer solution (4 mM CaCl₂/0.1 M Tris-HCl, pH 8.0). The sample solution was incubated for 15 hours at 4°C and then diluted 2-fold with distilled water. The resultant sample solution (9.6 mL) was purified by affinity chromatography. The affinity chromatography was carried out on a Benzamidine Sepharose 6B (Pharmacia) column (0.8 cm²× 12 cm) under the following conditions:
mobile phase:
   1) 2 mM CaCl₂/0.05 M Tris-HCl, pH 8.0;
   2) 2 mM CaCl₂/0.05 M Tris-HCl, pH 8.0 - 0.5 M NaCl;
   3) 2 mM CaCl₂/0.05 M Sodium Acetate, pH 4.25; and
   4) 2 mM CaCl₂/0.05 M Sodium Acetate, pH 3.25,
flow rate: 24 mL/hr, and
fraction size: 2 mL/fraction.
Each fraction was tested for absorbance at 280 nm (A₂₈₀) and enzyme activity. The enzyme assay was carried out as follows: To a small test tube was added 1.7 mL of 2 mM CaCl₂/0.05 M Tris-HCl, pH 8.0, and then 100 µL each of suitably diluted eluate samples. To the mixture was added 200 µL of 1 mM benzoyl arginine ethyl ether (BAEE) to initiate a reaction.
The time-course of reaction was observed by measuring the absorbance at 253 nm (A₂₅₃), provided that it was defined as 0.001/min.=1 BAEE Unit.

Potential active fractions Nos.80 to 93 were pooled together and subjected to dialysis against 1 mM hydrochloric acid. Next, after dialysis, the sample was lyophilized to give a dry product corresponding to cationic trypsin. Similarly, potential active fractions Nos.111 to 121 were pooled together and subjected to dialysis against 1 mM hydrochloric acid.
Next, after dialysis, the sample was lyophilized to give a dry product corresponding to anionic trypsin.

The anionic trypsin (20 mg) was stored at -80°C until further use.

Among the resultant cationic trypsin (17 mg), 7 mg of cationic trypsin sample was dissolved in 0.5 mL of 2 mM CaCl₂/0.05 M Tris-HCl, pH 8.0 - 0.5 M NaCl. The resulting sample solution was purified by affinity chromatography. The affinity chromatography was carried out on a Benzamidine Sepharose 6B (Pharmacia) column (0.8 cm²× 12 cm) under the following conditions:
mobile phase:
   1) 2 mM CaCl₂/0.05 M Tris-HCl, pH 8.0 - 0.5 M NaCl;
   2) 2 mM CaCl₂/0.05 M Sodium Acetate, pH 4.25; and
   3) 2 mM CaCl₂/0.05 M Sodium Acetate, pH 3.25,
flow rate: 24 mL/hr, and
fraction size: 2 mL/fraction.
Eluate fractions at the mobile phase: 2), were pooled together and subjected to dialysis against 1 mM hydrochloric acid. After dialysis, the sample was lyophilized to give a dry product (6.2 mg) which was stored at -80°C until further use. As a result of N-terminal amino acid sequence analysis of the sample, Ile-Val-Gly-Gly-Tyr-Thr was identified as its N-terminal sequence, identical with that of canine cationic trypsin. The N-terminal sequence corresponds to an Ile²⁴ to Thr²⁹ amino acid sequence of canine cationic trypsinogen having SEQ ID NO: 2 in the Sequence Listing.

SEQ ID NO: 1 in the Sequence Listing shows an amino acid sequence of canine anionic trypsinogen. The aforementioned sample for anionic trypsin was also subjected to N-terminal amino acid sequence analysis, leading to similar results.

### Example 2

### Preparation of Monoclonal Antibody

### (a) Preparation of Antibody-Producing Cells

Cationic trypsin prepared in Example 1 was used as an antigen. The antigen was diluted with phosphate-buffered saline (PBS, 0.1M, pH7.4) to bring the antigen concentration to 100 µg/100 µL. The antigen solution was mixed equivalently with a complete Freund's adjuvant to form an emulsion. A six-week old female Balb/c mouse was initially primarily immunized by administering intraperitoneally the emulsion at 100 µg/200 µL/animal. After an interval of 10 days to 2 weeks the animal was supplementally immunized. For the supplemental immunization, an antigen solution was prepared to bring the antigen concentration to 100 µg/100 µL. The resultant antigen solution was mixed equivalently with an incomplete Freund's adjuvant to form an emulsion which was then administered intraperitoneally to each initially immunized animal at 100 µg/200 µL/animal. Further, after an interval of 10 days to 2 weeks the supplementally immunized animal was immunized. If necessary, immunization can be repeated at these intervals. An antigen solution for final immunization was prepared to bring the antigen concentration to 100 µg/200 µL. The resultant antigen solution per se was administered intravenously to each immunized animal for final immunization. Next three days later, the spleen was taken out, and a spleen cell suspension was prepared.

Two mice were used for immunization. Similarly, mice can be immunized with anionic trypsin as an antigen.

### (b) Preparation of Antigen Polypeptides

For sequences specific to the N-terminal, central, or C-terminal area of canine cationic trypsin, the following sequences were selected from the amino acid sequence of canine cationic trypsin as described in SEQ ID NO: 2 in the Sequence Listing, and synthesized: 
(SEQ ID NO: 3)
Cys Leu Ile Ser Gly Trp Gly Asn Thr Gln Ser Ile Gly Gln Asn Tyr
Pro Asp Val Leu
 (corresponding to Sequence: Cys¹³⁹ to Leu¹⁵⁸ of SEQ ID NO: 2 in the Sequence Listing) 
(SEQ ID NO: 4)
Ile Val Gly Gly Tyr Thr Cys Ser Arg Asn Ser Val Pro Tyr Gln Val
Ser Leu Asn Ser
 (corresponding to Sequence: Ile²⁴ to Ser⁴³ of SEQ ID NO: 2 in the Sequence Listing) 
(SEQ ID NO: 5)
Leu Gln Gly Val Val Ser Trp Gly Ala Gly Cys Ala Gln Lys Gly Lys
Pro Gly Val Ser
 (corresponding to Sequence: Leu²¹⁰ to Ser²²⁹ of SEQ ID NO: 2 in the Sequence Listing)

These peptides were synthesized using a peptide synthesizer (Peptide Synthesizer 9600, MilliGen/Biosearch) with Fmoc-bop methods. The synthetic peptides were purified by high performance liquid chromatography using *µ* Bondasphere, C18 column (Waters).

Similarly, suitable peptides are selected from the amino acid sequences as described in SEQ ID NOs: 1 and 2 in the Sequence Listing, and synthesized.

### (c) Preparation of Polypeptide-BSA Conjugates

Each peptide was coupled with bovine serum albumin (BSA) via a cysteine residue to form an antigen-conjugate. BSA (19.6 mg) was dissolved in 1 mL of 0.1 M phosphate buffer, pH 7.0. Also, 2.22 mg of EMCS (N-(6-maleimidecaproyloxy)-succinimide) was dissolved in 48.3 µL of dimethylformamide. A mixture of the BSA solution and the EMCS solution was reacted at 30°C for 30 min., and then subjected to gel filtration using PD-10 (Pharmacia) equilibrated with a 0.1 M phosphate buffer, pH 7.0. The concentration of the resultant maleimido-coupled BSA was 6.22 mg/mL. Each synthetic polypeptide obtained in the above (b) was dissolved in a 0.1 M phosphate buffer, pH 7.0, then mixed with the maleimido-coupled BSA thus prepared (molar ratio of polypeptide: maleimido-coupled BSA = 50: 1). For example, the polypeptide (1788 nmol) was mixed with the maleimido-coupled BSA (20 to 40 nmol). A total volume of the mixture was adjusted to 1 mL and incubated at 4°C for 20 hours to form a BSA-polypeptide conjugate. The protein concentration of the resultant BSA-polypeptide conjugate ranged from 4 to 6 mg/mL. Each BSA-polypeptide conjugate was diluted with a 0.1 M phosphate buffer, pH 7.0, to 200 µg/150 µL, poured to each tube (150 µL per tube) and cryopreserved at -80°C.

### (d) Preparation of Antibody-Producing Cells with BSA-Polypeptide Conjugates

An eight-week old female Balb/c mouse was initially immunized by administering intraperitoneally 200 µg of each BSA-polypeptide conjugate (prepared in the above (c) step) together with complete Freund's adjuvants. Nineteen and thirty-four days later, 200 µg of the BSA-polypeptide conjugate dissolved in a 0.1 M phosphate buffer, pH7.5, was administered intraperitoneally to the initially immunized mouse for booster. Further 69 days later, 200 µg of the BSA-polypeptide conjugate solution was administered intravenously to the mouse for final immunization. Next three days later, the spleen was taken out, and the spleen cell suspension was prepared.

Two mice were used for immunization with each BSA-polypeptide conjugate.

### (e) Cell Fusion

(1) The following materials and methods were used:
   RPMI-1640 medium:
      To RPMI-1640 (Flow Lab.) were added sodium bicarbonate (24 mM), sodium pyruvate (1 mM), penicillin G potassium (50 U/mL), and amikacin sulfate (100 µg/mL), and the mixture was adjusted pH to 7.2 with dry ice, sterilized and filtered through a 0.2 µm Toyo Membrane Filter.
   NS-1 medium:
      To the above RPMI-1640 medium was added filter sterilized fetal calf serum (FCS, M. A. Bioproducts) until a concentration of FCS reached 15% (v/v).
   PEG 4000 solution:
      To RPMI-1640 medium was added polyethylene glycol 4000 (PEG-4000, Merck & Co.) until a concentration of PEG 4000 reached 50% (w/w). Thus, the serum-free solution was prepared.
      Cell fusion using 8-azaguanine-resistant myeloma SP-2 cells (SP-2/0-Ag14) was carried out by slightly modified methods according to Oi et al. techniques disclosed in "Selected Method in Cellular Immunology, p.351 to 371 (ed. B. B. Mishell and S. N. Shiigi), W. H. Freeman and Company (1980)".
(2) The respective nucleated spleen cells (viable cell ratio: 100%) prepared in the foregoing (a) or (d) were fused with myeloma cells (viable cell ratio: 100%) at a ratio of 5 : 1 according to the following procedure:

Each canine trypsin-immunized spleen cell suspension and the myeloma cells were washed respectively with a RPMI 1640 medium followed by resuspending in the same medium. For fusion, 1 x 10⁸ to 6 x 10⁸ nucleated spleen cells and 3 x 10⁷ to 2 x 10⁸ myeloma cells were mixed together. The cell suspension was then pelleted by centrifugation and the supernatant was completely aspirated off. To the cell pellet was added a PEG 4000 solution (RPMI 1640 medium containing 50% (w/v) polyethylene glycol 4000) pre-warmed to 37°C dropwise for 1 min. (the volume of the PEG 4000 solution to be added was determined to give 3 x 10⁷ myeloma cells/mL), and stirred for 1 min, to allow the cells to be resuspended and dispersed.
Next, after 37°C pre-warmed RPMI 1640 medium was added dropwise for 2 minutes (RPMI 1640 medium : 50% PEG 4000-containing RPMI 1640 medium already added = 2 : 1 in volume), the same medium was added dropwise within 2 to 3 minutes with stirring (RPMI 1640 medium : 50% PEG 4000-containing RPMI 1640 medium = 7 : 1 in volume) to allow the cells to be dispersed. This cell dispersion was centrifuged, and the supernatant fluid was completely aspirated off. To the cell pellet was added 37°C pre-warmed NS-1 medium quickly until a concentration of myeloma cells reached 3 x 10⁶ cells/mL, and a large cell mass was carefully dispersed by pipetting. Next, the cell suspension was diluted with the same medium, and 6.0 x 10⁵ cells/well was plated on each well of a polystyrene 96-well microtiter tray. The cell-containing microwell was incubated at 37°C under a 100% humidified atmosphere containing 7% CO₂/93% air.

### (f) Selective Growth of Hybridomas in Selection Medium

(1) Media to be used were as follows:
   HAT medium: To NS-1 medium as described in the foregoing (e)-(1) was added further hypoxanthine (100 µM), aminopterin (0.4 µM), and thymidine (16 µM).
   HT medium: The medium has the same composition as the foregoing HAT medium except that aminopterin was excluded.
(2) Next day (first day) from culture initiation of the foregoing (e), two drops of HAT medium (approximately 0.1 mL) was added to the cells with a Pasteur pipette. On the 2nd, 3rd, 5th, and 8th days, a half of the medium (approximately 0.1 mL) was replaced with fresh HAT medium. On the 11th day, a half of the medium was replaced with fresh HT medium. On the 14th day, positive wells were examined by solid phase-antibody binding test (enzyme-linked immunosorbent assay; ELISA) for all wells wherein the growth of hybridomas was visually recognized.

First, polystyrene 96-well plates were coated with anionic or cationic trypsin purified in Example 1. For example, the concentration of trypsin was adjusted to 1 µg/mL and the trypsin solution was added to each well at 100 µL/well. Each well was then washed with PBS containing 0.05% Tween 20 (trade name) for washing to remove unadsorbed antigens.
Further uncoated sites of each well were blocked with 1% BSA. After rinsing, each antigen-coated well received 100 µL of a supernatant fluid from the hybridoma well in which hybridomas were grown. The well was allowed to stand at 37°C for approximately 1 hour. After washing, horseradish peroxidase (HRP)-labeled goat anti-mouse immunoglobulin (Cappel Lab.) was added as a second antibody to the antigen-coated well, and the well was further allowed to stand at 37°C for approximately 1 hour. Next, after washing, to the well was added substrates, hydrogen peroxide and 2,2'-azino-bis(3-ethylbenzthiazoline-6-sulfonic acid), and OD readings at 405 nm were obtained by a microplate OD reader (MRP-A4, Tosoh).

### (g) Cloning of Hybridomas

Hybridomas in the wells positive against trypsin, obtained in the foregoing (f), were cloned by limiting dilution to establish monoclones.

That is, a cloning medium containing, as feeder cells, 10⁷ mouse thymocytes per 1 mL of NS-1 medium was prepared. Into a 96-well microtiter tray was plated hybridomas at a cell density of 5, 1, or 0.5 cells per well, respectively, with dilutions wherein the 5, 1, or 0.5 hybridoma cells per well was plated to 36, 36, and 24 wells, respectively.
On the 5th and 12th days, about 0.1 mL of NS-1 medium was added to all the wells. Approximately two weeks later from the initiation of cloning, ELISA as described in the above (f) was conducted for groups wherein the sufficient growth of hybridomas was visually recognized and the rate of colony formation-negative wells is 50% or more. In cases where all the examined wells were negative, 4 to 6 wells each containing 1 colony were selected from antibody-positive wells, and recloned. Finally, hybridoma cells each producing an anti-trypsin antibody were obtained.

### (h) Determination of Class and Sub-class for Monoclonal Antibody

To microtiter plates on which trypsin was coated according to ELISA as described herein above, was added each supernatant of the hybridomas obtained in the above (g). Next, after PBS washing, iso-type specific rabbit anti-mouse IgG antibodies (Zymed Lab.) were added. After PBS washing, horseradish peroxidase-labeled goat anti-rabbit IgG (H+L) was added, and visualization was carried out with hydrogen peroxide and 2,2'-azino-di(3-ethylbenzothiazolinic acid). As a result, the class and sub-class were determined.

Each clone No. assigned to each monoclonal antibody-producing hybridoma clone which is produced via immunization with trypsin, obtained in Example 1, together with its subclass type, is shown in Tables 1 and 2.

**TABLE 1**

| Clone No. | Subclass | Avidity with Cationic Trypsin | Avidity with Anionic Trypsin |
|---|---|---|---|
| 004-301 | γ1/κ | + + + + | + + + |
| 004-312 | γ1/κ | + + + + | + + + |
| 004-203 | γ1/κ | + + + + | - |
| 004-214 | γ1/κ | + + + + | - |
| 005-201 | γ1/κ | + + + + | - |
| 005-202 | γ1/κ | + + + + | ± |
| 005-203 | γ1/κ | + + + + | + |
| 005-204 | γ1/κ | + + + | ± |
| 005-205 | γ1/κ | + + + + | + |
| 005-206 | γ1/κ | + + + | - |
| 005-208 | γ1/κ | + + + + | - |
| 007-202 | γ1/κ | + + + | - |
| 007-203 | γ1/κ | + + + | - |
| 007-205 | γ1/κ | + + + | - |
| 007-206 | *µ*/κ | + + + | + + + |
| 007-207 | γ1/κ | + + + | - |
| 007-209 | γ1/κ | + + + | - |

**TABLE 2**

| Clone No. | Subclass | Avidity with Cationic Trypsin | Avidity with Anionic Trypsin |
|---|---|---|---|
| 008-202 | γ1/κ | + + + | - |
| 008-204 | γ1/κ | + + + | - |
| 008-205 | γ1/κ | + + + | - |
| 008-206 | γ1/κ | + + + | - |
| 008-207 | γ1/κ | + + + | - |
| 009-201 | γ1/κ | + + + + | - |
| 009-202 | γ1/κ | + + + + | - |
| 009-303 | γ1/κ | + + + + + | - |
| 009-204 | γ1/κ | + + + + | - |
| 009-205 | γ1/κ | + + + | - |
| 009-206 | γ2a/κ | + + + | - |
| 009-207 | γ1/κ | + + + | - |
| 009-209 | γ1/κ | + + + | ± |

The aforementioned monoclonal anti-canine trypsin antibody-producing hybridomas:
Clone No. 004-203 (Accession Number FERM P-16914) and
Clone No. 005-201 (Accession Number FERM P-16915) have been deposited as from July 28, 1998 with NIBH.
The original deposit of the hybridoma: 004-203 has been transferred to one under the Budapest Treaty by a request dated July 30, 1999 and is on deposit with the Accession Number FERM BP-6808 under the terms of the Budapest Treaty at NIBH.

Further, the aforementioned monoclonal anti-canine trypsin antibody-producing hybridomas:
Clone No. 008-207 (Accession Number FERM P-17290) and
Clone No. 009-303 (Accession Number FERM P-17291) have been deposited as from March 9, 1999 with NIBH.

The hybridoma 008-207 and 009-303 original deposits have been transferred to those under the Budapest Treaty by requests dated July 26, 1999 and are on deposits with the Accession Numbers FERM BP-6795 and FERM BP-6796, respectively, under the terms of the Budapest Treaty at NIBH.

### (i) Hybridoma Cultivation and Purification of Monoclonal Antibodies

Each hybridoma cell thus obtained was grown in NS-1 medium to afford monoclonal antibodies with a concentration of 10 to 100 µg/mL in the culture supernatant.

Alternatively, 10⁷ hybridoma cells thus obtained were administered intraperitoneally to a mouse (inbred Balb/c mouse, 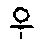 , six-week old) primed intraperitoneally with Pristane 1 week prior to hybridoma injection, and 1 to 2 weeks later an ascites containing 4 to 7 mg/mL monoclonal antibody was recollected. After the obtained ascitic fluid was salted out with 40% ammonium sulfate saturation, 16 mL of the ascitic fluid (for example, antibody 004-203) was diluted 2-fold with phosphate-buffered saline (pH7.4) to which was added 25.2 mL of saturated ammonium sulfate solution to achieve a 40% saturated final concentration. The resultant mixture was incubated at 4°C overnight and then centrifuged at 13,500 rpm for 30 min. The pellet was resuspended with distilled water and dialyzed against 60mM NaCl/0.03M Tris-HCl(pH8.0). The dialyzed monoclonal antibody was passed through a DEAE-Sepharose (Pharmacia) column. Unabsorbed fractions were collected at 3mL/fraction and dialyzed against phosphate-buffered saline (pH7.4). The antibody (32 mL) was obtained at 1.38 mg/mL and stored at -80°C. IgG class antibodies can also be adsorbed on protein A Affi-Gel (Bio-Rad), followed by elution with 0.1 M citrate buffer, pH 5.0 to afford purified forms.

### Example 3

### Immunoreactivity against Canine Trypsin

Each monoclonal antibody (MAb) prepared in Example 2 was tested for avidity (immunoreactivity) with cationic trypsin and anionic trypsin as follows:

Cationic trypsin and anionic trypsin used herein were isolated and purified from a canine pancreas according to Example 1.

To 96-well microtiter plates (costar) was added cationic trypsin or anionic trypsin and the plates were incubated at 4°C overnight for coating. To each well was added 300 µL of 30mM phosphate buffer (pH7.0, containing 1% BSA, 0.1M sodium chloride and 10mM EDTA; buffer A) and the wells were stored at 4°C. To the well was added 1 µg/mL of anti-trypsin MAb and the mixture was incubated at 37°C for 1 hour, followed by addition of anti-mouse IgG· IgM· IgA-HRP Ab (KPL) at 1 µg/mL. The resultant wells were incubated at 37°C for 1 hour. Next, substrates, H₂O₂ and 2,2'-azino-bis(3-ethylbonzthiazoline-6-sulfonic acid), were added to each well, and OD readings at 405 nm were conducted by a microplate OD reader (MRP-A4, Tosoh, Japan). The immunoreactivity was scored as - to + + + + + according to absorbance as follows:

| | |
|---|---|
| - | 0 to not more than 0.1, |
| ± | 0.1 to not more than 0.2, |
| + | 0.2 to not more than 0.4, |
| + + | 0.4 to not more than 0.6, |
| + + + | 0.6 to not more than 0.8, |
| + + + + | 0.8 to not more than 1.0, and |
| + + + + + | 1.0 or more. |

The results are shown in Tables 1 and 2.

### Example 4

### Sandwich Assay

Sandwich assay systems capable of specifically detecting and/or measuring canine trypsin can be composed by a combination of suitable two antibodies selected from monoclonal anti-trypsin antibodies as prepared in Example 2, according to techniques as disclosed herein below. An example is a combination of MAb clone No.004-203 and MAb clone No.005-201. Further, MAb clone No.004-203 can be used in combination with MAb clone No.005-208. Similarly, MAb clone No.004-214 can be used in combination with either MAb clone No.005-201 or No.005-208.

Each reaction buffer composition and reaction condition can vary depending on assay purposes, including shortening, extension, etc. Standard antigen trypsin can be isolated and purified from pancreas, tissue supernatants derived therefrom or pancreatic juice.

For example, 5 mL of 11.7 mg/mL MAb 009-303 was used. The MAb 009-303 was dialyzed against 0.5M NaCl/0.1M Na-borate (pH8.0) and diluted to bring the MAb concentration to 0.7 mg/mL (total volume: 71.3 mL). CNBr-Activated Sepharose 4B (Pharmacia), 5 g, was swollen in 50 mL of 1mM HCl for 30 min., filtered on an Advantec 2 filter paper (Advantec), and then washed with 800 mL of 1mM HCl and 100 mL of 0.5M NaCl/0.1M Na-borate (pH8.0) successively. To the resultant resin was added 71.3 mL of the diluted MAb 009-303 solution prepared hereinabove to form a suspension which was allowed to react at 4°C overnight and further shaken at room temperature for 3 hours. The immobilized antibody was filtered on an Advantec 2 filter paper, washed with 500 mL of 0.5M NaCl/0.1M Na-borate (pH8.0) and then suspended in 0.2M Tris-HCl(pH8.0). The suspension was allowed to react at room temperature for 2 hours. The immobilized antibody was filtered on an Advantec 2 filter paper, and then washed successively with 500 mL of 0.5M NaCl/0.1M Na-borate (pH8.0) and 500 mL of 0.5M NaCl/0.1M Na-acetate (pH4.0). This two-buffer cycle was repeated 4 times. Further, the immobilized antibody was washed with 300 mL of 0.2M glycine-HCl(pH2.3). After the buffer was replaced with 0.1% NaN₃/0.1M Na-phosphate (pH7.0), the immobilized antibody was stored. For the obtained MAb 009-303-coupled resin, its calculated IgG binding efficiency is 92.8%.

### Affinity Purification

Canine pancreas was homogenized, and then fractionated with ammonium sulfate. The fractionated sample was purified by affinity chromatography with a MAb 009-303 column.

After the buffer was replaced with 2mM CaCl₂/0.05M Tris-HCl(pH7.5), the MAb 009-303-immobilized resin obtained hereinabove was packed in a column. The sample (10 mg) was dissolved in 5 mL of 2mM CaCl₂/0.05M Tris-HCl (pH7.5) and subjected to purification. After flow-through fractions were collected, the column was subjected to an elution with 2mM CaCl₂/0.2M glycine-HCl(pH2.5). Every 4 mL fraction was collected in a test tube wherein 1 mL of 2mM CaCl₂/3M Tris-HCl (pH7.5) was placed in advance. Peak fractions were pooled to afford 2.59 mg of cationic trypsin.

Purification of trypsin can also be achieved by various combinations of other affinity chromatographic techniques. Further, trypsin can be purified by ion chromatography, gel filtration, etc.

### (a) Preparation of Gold Colloid (Gold Sol) Labels

Distilled water (200 mL) was heated to boiling point. To the boiled water was added 4 mL of 1% HAuCl₄ followed by addition of 1% trisodium citrate (4 mL). The solution was heated at the boiling point until the initial blue color of the solution turned to reddish purple and then cooled to room temperature. The resultant gold colloid solution was filtered through a 0.2 µm filter. The obtained gold sols were used as labels.

The resultant gold colloids were titrated as follows: Gold colloids were taken at an amount of 1 mL and placed into a test tube to which was added a series of antibody solution to bring the antibody concentration to 0, 1, 2, 3, ..., or 15 µg, and stirred for 2 min. followed by addition of 10% aqueous NaCl solution (100 *µ* L). The mixture was stirred for 5 min. and A₅₃₀ was measured.

### (b) Preparation of Labeled Antibody

To 50 mL of the gold colloid solution (adjusted to pH 9) prepared by the aforementioned method was added 5 mL of anti-trypsin MAb (1 mg/mL, dialyzed against 2 mM of Borax, Wako Pure Chemical Industries, Ltd.) and the mixture was stirred for 5 min. Next, 5.5mL of 2 mM Borax containing 10% bovine serum albumin (BSA) was added to the mixture. The resultant mixture was centrifuged at 10,000 rpm for 30 min. to separate a precipitate. After addition of 2 mM Borax containing 1% BSA, the precipitate was dispersed and then centrifuged at 10,000 rpm for 30 min. To the precipitate thus obtained was added 10 mM TBS (Wako Pure Chemical Industries, Ltd., pH8.2) containing 1% BSA and the mixture was dispersed. The resultant labeled antibody was filtered through a 0.2 µm filter and used for assay.

### (c) Preparation of Monoclonal Antibody-Coupled Carrier

Anti-trypsin MAb was dissolved in 0.01M phosphate buffer (pH7.5) to bring the MAb concentration to 4 mg/mL. Polyclonal anti-mouse IgG antibodies were dissolved in 0.01M phosphate buffer (pH7.5) to bring the polyclonal antibody concentration to 4 mg/mL. This MAb solution was applied to one site on an SNHF membrane (Millipore) for antibody-binding at a dose of 1 µL/line per 1 assay. Similarly, the polyclonal antibody solution was applied to one site different from the MAb solution-applied site on the SNHF membrane at a dose of 1 µL/line per 1 assay. The membrane to which the antibody solution adhered was dried at 50°C for 30 min., dipped successively into 10 mM carbonate-phosphate buffer (pH7.5) containing 1% BSA for 30 min. and 5 mM carbonate-phosphate buffer (pH7.5) containing 0.05% Tween 20 for 30 min, and then dried at 50°C for 30 min.

### (d) Production of Polyclonal Anti-Mouse IgG Antibody

Purified mouse IgG (Jackson Immuno Research) was employed as an antigen to raise a polyclonal anti-mouse IgG antibody.

The antigen was dissolved in phosphate-buffered saline (PBS, 0.1M, pH7.4) to bring the antigen concentration to 1 mg/100 µL. The antigen solution was admixed with an equal amount of complete Freund's adjuvant to form an emulsion. The emulsion was administered subcutaneously to the back site of a Japanese white rabbit (body weight: 2 kg) at a dose of 1 mg/200 µL/animal for initial immunization. The animal was supplementally immunized at an interval of 10 days to 2 weeks. For this supplemental immunization, the antigen solution was adjusted to 1 mg/100 µL, admixed with an equal amount of incomplete Freund's adjuvant to form an emulsion which was administered subcutaneously to the back site of the initially immunized rabbit (dose: 1 mg/200 µL/animal).

Further, the animal was supplementally immunized at an interval of 10 days to 2 weeks. If necessary, immunization can be repeated at these intervals. The antigen solution was prepared to bring the antigen concentration to 1 mg/200 µL, and, without any modification, administered intravenously to the animal for final immunization. Three days later, the animal was bled from the ear vein to collect 60 mL of blood. The collected blood was stored at 4°C overnight, and then centrifuged at 3000 rpm for 20 min. to give 30 mL of supernatant (serum). To this serum was added an equal amount (30 mL) of PBS (pH7.4) and 30 mL of saturated ammonium sulfate solution to bring the final ammonium sulfate concentration to 33%. The mixture was allowed to stand at 4°C overnight, and then centrifuged at 13000 rpm for 30 min. to separate a precipitate. To the resultant precipitate was added distilled water, the mixture was suspended and dialyzed against 0.03M Tris-HCl(pH8.3)-0.05M NaCl. The dialyzed sample was applied to an ion exchange chromatography on DEAE-Sepharose (Pharmacia, mobile phase: 0.03M Tris-HCl(pH8.3)-0.05M NaCl). Flow-through fractions were collected to afford 27.5 mL of polyclonal anti-mouse IgG antibody (antibody concentration: 4.56 mg/mL), stored at -80°C.

### (e) Preparation of Labeled Antibody-Conjugated Pad

Conjugate Pad (Pall Gelman) was dipped into 10 mM carbonate-phosphate buffer (pH7.5) containing 1% BSA for 30 min., then into 5 mM carbonate-phosphate buffer (pH7.5) containing 0.05% Tween 20 for 30 min., and dried at 50°C for 30 min. The dried Conjugate Pad was impregnated with gold colloid-labeled antibodies diluted with 2 mM Borax containing 0.05% Tween 20, 1% BSA and 5% sucrose, and dried at 50°C for 30 min.

### (f) 1 Step Sandwich Assay

The parts prepared in the foregoing (c) and (e) were fabricated to form a lateral flow device which was applied to an assay. Trypsin purified by affinity chromatography was used as a standard antigen. The purified trypsin was dissolved in 10 mM TBS (pH7.5) containing 1% BSA to give standard antigen dilutions each with a known concentration. The resultant standard antigen dilution was applied as a test sample to the lateral flow device as constructed above at an amount of 50 *µ* L. It was found that lines produced by gold colloid labels were observable in all trypsin concentration ranges from 5 to 10 ng/mL and therefore detection was performable. Table 3 shows the results wherein the membrane-coupled antibody is used in combination with the gold colloid-labeled antibody in the sandwich form. The assay runs wherein MAb clone No.005-201 was used for the gold colloid-labeled antibody and MAb clone No.004-203 for the membrane-coupled antibody gave better detection sensitivity. The sensitivity increased to 0.5 to 1 ng/mL when the gold colloid-labeled antibody 009-303 was used in combination with the membrane-coupled antibody 008-207.

In the lateral flow device, an anti-trypsin MAb-applied site is set as a sample line (capture line), and a polyclonal anti-mouse IgG antibody-applied site as a control line. In the device with the highest sensitivity of 0.5 to 1 ng/mL, the spot of gold colloids was visualized on both the sample and control lines. To satisfy kits clinically required, the amount of membrane carrier-coupled antibodies varied whereby, for the application of 2 ng/mL of standard trypsin antigen, the gold colloid spot was invisible on the sample line but visualized on the control line only. When 5 ng/mL thereof was applied, the gold colloid spot was visualized on both the sample and control lines. Thus, its sensitivity is controllable and it is possible to set a suitable cut off value.

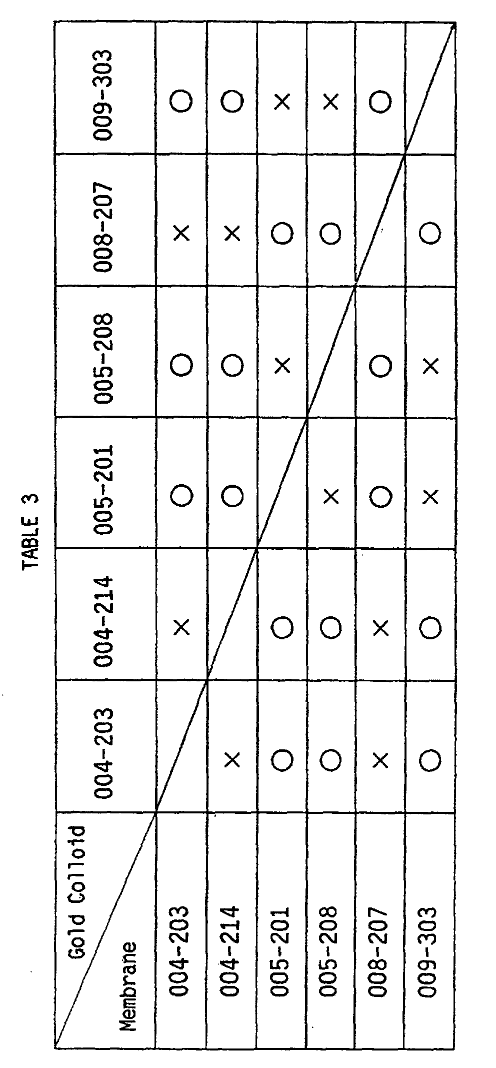

### (g) Competitive 1 Step Sandwich Assay

Purified trypsin was dissolved in 0.1M phosphate buffer (pH7.5) to bring the trypsin concentration to 2 mg/mL. This trypsin antigen solution was applied on a competitive antigen binding membrane, SNHF membrane, on which the trypsin antigen would adhere at 1 µL/line per assay. The membrane to which the antigen solution adhered was dried at 50°C for 30 min., dipped into 10 mM carbonate-phosphate buffer (pH7.5) containing 1% BSA for 30 min. and 5 mM carbonate-phosphate buffer (pH7.5) containing 0.05% Tween 20 successively, and dried 50°C for 30 min.

The parts thus prepared were fabricated with the parts prepared in the foregoing (e) to form a competitive assay lateral flow device which was applied to an assay. Similarly to the above (f), standard antigen dilutions were prepared each with a known concentration. The resultant standard antigen dilution was applied as a test sample to the lateral flow device as constructed above at an amount of 50 µL. It was found that lines produced by gold colloid labels disappeared in all trypsin concentration ranges from 250 to 500 ng/mL and therefore detection was performable. The assay runs wherein MAb clone No.005-201 was used for the gold colloid-labeled antibody gave better detection sensitivity.

### Example 5

### Western Blotting with Anti-Canine Trypsin MAb

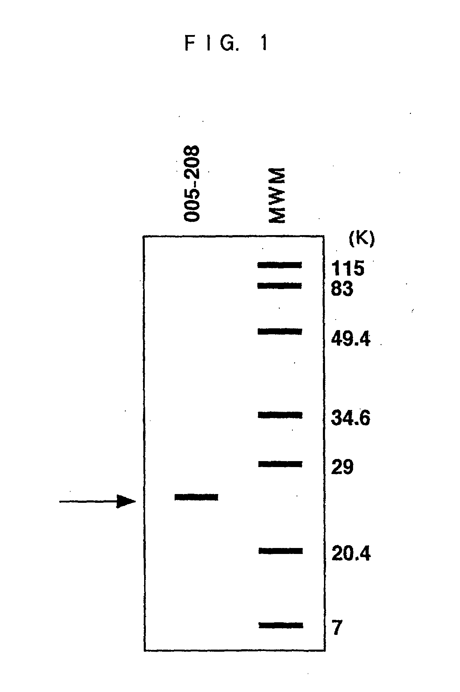

A canine cationic trypsin sample (2.5 µg/lane) purified from canine pancreas according to Example 1 was applied to SDS-PAGE (10% total acrylamide) under a reducing condition. Then the sample was transferred onto a nitrocellulose filter. Next, it was contacted with anti-canine trypsin MAb clone No.005-208 (1 mg/mL) and visualization was conducted by the avidin-biotin-peroxidase complex method according to Nomura, H., et. al., Cancer Res., 55, 3263-3266 (1995) (Western blotting). The results are shown in FIG. 1. It was verified that anti-canine trypsin MAb (clone No.005-208) was immunoreactive to canine trypsin samples (cationic trypsin).

### Example 6

### Sandwich EIA

### (a) Preparation of Enzyme-Labeled Antibody (IgG-HRP Conjugate)

### (1) Preparation of SH-Labeled IgG

Anti-canine trypsin IgG-HRP conjugates were prepared according to the Ishikawa et al. method as described in J. Immunoassay, 4, 209 to 327, 1983.

The monoclonal antibody (IgG) obtained in accordance with the present invention and recognized to be immunoreactive to canine trypsin was dialyzed against about 0.1M phosphate buffer (pH about 6.5). To the dialyzed MAb solution was added a solution of S-acetylmercaptosuccinic anhydride in DMF (IgG:S-acetylmercaptosuccinic anhydride molar ratio, 1: about 100) and the mixture was incubated at about 30°C for about 30 min. Next, to the mixture was added about 100 µL of ca. 0.1M Tris-HCl buffer (pH about 7.0), about 10 µL of ca. 0.1M EDTA solution (pH about 6.0), and about 100 µL of ca. 1M hydroxylamine solution (pH about 7.0), the mixture was allowed to stand at ca. 30°C for about 5 min. and then subjected to gel filtration on Sephadex G-25 equilibrated with ca. 0.1M phosphate buffer, pH about 6.0 containing ca. 5mM EDTA to afford SH-labeled anti-canine trypsin IgG fractions.

### (2) Preparation of Maleimide-Conjugated HRP

HRP was dissolved in 28.3 µL of 0.1 M phosphate buffer (pH7.0) to bring the HRP concentration to 14.6 mg/mL. To the HRP solution was added 5.6 mg of EMCS dissolved in 56 µL of DMF (EMCS:HRP molar ratio, 25:1). The mixture was incubated at 30°C for 30 min. and subjected to gel filtration on a Sephadex G-25 column equilibrated with 0.1 M phosphate buffer (pH6.0) to collect maleimide-conjugated HRP fractions (9.2 mg).

### (3) Preparation of IgG-HRP Conjugate

The SH-labeled IgG (about 1 mole) prepared in the foregoing (1) was admixed with the maleimide-conjugated HRP (about 5 moles) obtained in the foregoing (2), allowed to stand at about 4°C for about 20 hours, and then subjected to gel filtration on an Ultrogel AcA 44 column equilibrated with ca. 0.1M phosphate buffer (pH about 6.5) to collect anti-canine trypsin IgG-HRP conjugate fractions which were stored at about 4°C.

### (b) Preparation of Enzyme-Labeled Antibody (Fab'-HRP Conjugate)

### (1) Preparation of Fab'

Purified MAb 009-303 (IgG, 96 mg) obtained according to the present invention was dissolved in 7 mL of 0.1 M acetate buffer (pH4.2) containing 0.1M NaCl and the resultant solution was digested with pepsin as follows:

The above IgG solution was admixed with 700 µL (1.92 mg) of pepsin (enzyme: antibody ratio, 2% w/w). The digestion was carried out at 37°C for 24 hours and stopped with the addition of 3M Tris-HCl buffer (1.28 mL, pH7.5) for adjusting the pH of the digested mixture to 7.0. F(ab')₂ were collected by gel filtration on an Ultrogel Ac A44 column equilibrated with 0.1M phosphate buffer (pH7.0). As a result, 16 mg of F(ab')₂ was obtained. Next, 8 mg of this F(ab')₂ product was dialyzed against 0.1M phosphate buffer (pH6.0) containing 5mM EDTA. Aminoethanethiol hydrochloride was added to the dialyzed F(ab')₂ solution until the final aminoethanethiol concentration reached to 10 mM. The fragments were reduced at 37°C for 90 min. Fab' fractions were collected by gel filtration on an Ultrogel Ac A44 column equilibrated with 0.1M phosphate buffer (pH6.0) containing 5mM EDTA. As a result, 4.7 mg of Fab' was obtained.

### (2) Preparation of Fab'-HRP Conjugate

The Fab' (4 mg) prepared in the foregoing (1) was mixed with maleimide-conjugated HRP (3.5 mg) from the fraction obtained in the foregoing (a)(2) to form an equimolar mixture. The resultant mixture was diluted with 0.1M phosphate buffer (pH6.0) containing 5mM EDTA to bring the total volume to 1.2 mL until the final Fab' and maleimide-conjugated HRP concentrations reached to 50 µM, respectively. The resultant mixture was incubated at 4°C for 20 hrs, followed by blocking of unreacted thiol groups with 8.87µL of 0.1M N-ethyl maleimide (N-ethyl maleimide: Fab' molar ratio, 10:1). The mixture was subjected to gel filtration on an Ultrogel Ac A44 column equilibrated with 0.1M phosphate buffer (pH6.5) to collect Fab'-HRP conjugate fractions (60 µg/mL, 17 mL). The labeled Fab' fraction supplemented with 0.1% BSA and 0.001% chlorhexidine was stored at -80°C.

### (c) Preparation of Monoclonal Antibody-Coated Carrier

Purified MAb 004-203 obtained according to the present invention was dissolved in 0.1 M phosphate buffer (pH7.5) containing 0.1% sodium azide to bring the MAb concentration to 100 µg/mL according to the Ishikawa et al. method as described in J. Immunoassay, 4, 209 to 327, 1983.

This MAb solution was added to a 96-well microplate at 100 µL per well and allowed to stand at 4°C for 24 hours. Next, the MAb solution was removed from each well to which was then added 30mM phosphate buffer containing 1% BSA, 0.1M sodium chloride and 10mM EDTA (pH7.0, buffer A) at an amount of 300 µL. The resultant wells were stored at 4°C. Upon use, the well was washed 3 times with 10mM phosphate buffer containing 0.1M sodium chloride (pH7.0, washing buffer) and then employed.

### (d) Exploration of 1 Step Sandwich EIA System

Canine trypsin was diluted with fetal bovine serum (FBS) and added to each well of a 96-well microplate at an amount of 20 µL. Enzyme-labeled antibodies prepared from each monoclonal antibody were diluted with 0.05M phosphate buffer containing 0.5% BSA (pH8.0, buffer B) to bring the enzyme-labeled antibody concentration to 100 ng/mL and then added to each well of the aforementioned microplate at an amount of 100 µL to mix the canine trypsin with the enzyme-labeled antibody. The resultant mixture was added at an amount of 100 µL to each well of the antibody-coated plate prepared from each monoclonal antibody in the foregoing (c), incubated at 37°C for 1 hour, and then washed 3 times with the washing buffer. Next, 2,2'-azino-bis(3-ethylbenzthiazoline-6-sulfonic acid) (0.1 mg/mL) dissolved in 0.1M citrate-phosphate buffer (pH4.9) containing 0.02% hydrogen peroxide was added to each well at 100 µL per well, and incubated at 37°C for 50 min. The reaction was stopped by adding 100 µL of 2N sulfuric acid to each well. These reaction mixtures were measured using a microplate reader (MPR-A4, Tosoh) for absorbance at 405 nm (A₄₀₅).

The assay was also conducted using any combination of antibodies selected from those listed in, for example, Table 3 for the solid phase antibody and labeled antibody as used herein. Duplicate tests of the same sample are set within a single assay.

### (e) 1 Step Sandwich EIA

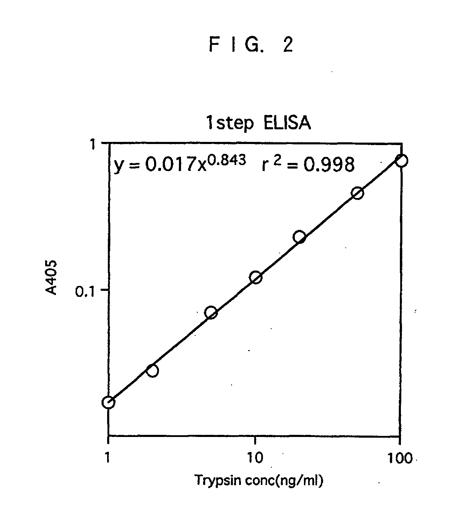

Canine trypsin (standard antigen) dilutions or test samples containing canine trypsin were prepared using FBS and added to each well of a 96-well microplate at an amount of 20*µ* L. Next, the enzyme-labeled antibody prepared in the foregoing (a) and (b) was diluted with 0.05M phosphate buffer containing 0.5% BSA (pH8.0, buffer B) to bring the enzyme-labeled antibody concentration to 100 ng/mL, and added to each well of the aforementioned microplate at an amount of 100 µL. After mixing, this mixture was added to each well of the antibody-coated plate prepared in the foregoing (c) at an amount of 100 µL, incubated at 37°C for 1 hour, and washed 3 times with the washing buffer. Next, 2,2'-azino-bis(3-ethylbenzthiazoline-6-sulfonic acid) (0.1 mg/mL) dissolved in 0.1M citrate-phosphate buffer (pH4.9) containing 0.02% hydrogen peroxide was added to each well at 100 µL per well, and incubated at 37°C for 50 min. The reaction was stopped by adding 100 µL of 2N sulfuric acid to each well. These reaction mixtures were measured using a microplate reader for absorbance at 405 nm (A₄₀₅). The canine trypsin concentration in test samples was determined by reference to a standard antigen dose-response curve. The A₄₀₅ increases straight depending on a raise in canine trypsin concentration for standard antigen samples (FIG. 2).

### (f) 2 Step Sandwich EIA

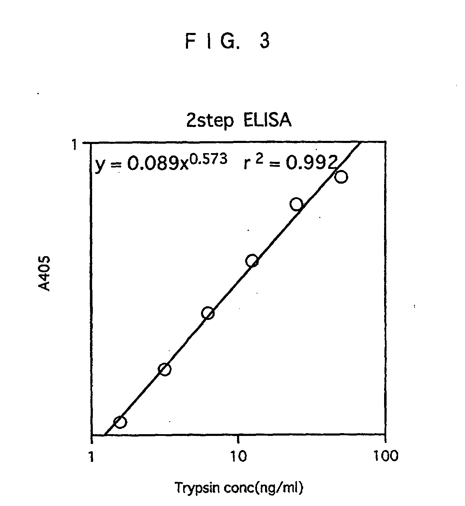

Canine trypsin (standard antigen) dilutions or test samples containing canine trypsin were prepared using FBS and added to each well of a 96-well microplate at an amount of 20 µL. Next, the buffer B was added to each well at an amount of 100 µL. After mixing, this mixture was added to each well of the antibody-coated plate prepared in the foregoing (c) at an amount of 100 µL, incubated at 37°C for 1 hour, and washed 3 times with the washing buffer. Next, the enzyme-labeled antibody, IgG-HRP, prepared in the foregoing (a) was diluted with the buffer B to bring the IgG-HRP concentration to 100 ng/mL, added to each well of the aforementioned plate at an amount of 100 µL, incubated at 37°C for 1 hour, and washed 3 times with the washing buffer. Next, 2,2'-azino-bis(3-ethylbenzthiazoline-6-sulfonic acid) (0.1 mg/mL) dissolved in 0.1M citrate-phosphate buffer (pH4.9) containing 0.02% hydrogen peroxide was added to each well at 100 µL per well, and incubated at 37°C for 30 min. The reaction was stopped by adding 100 µL of 2N sulfuric acid to each well. These reaction mixtures were measured using a microplate reader for absorbance at 405 nm (A₄₀₅). The canine trypsin concentration in test samples was determined by reference to a standard antigen dose-response curve. The A₄₀₅ increases straight depending on a raise in canine trypsin concentration for standard antigen samples (FIG. 3).

### (g) Test for Reproducibility

Assay tests were carried out for simultaneous and day-to-day reproducibility according to the method as described in the foregoing (e). The monoclonal antibody-coated carrier as mentioned in the foregoing (c) was employed for the solid phase antibody and Fab'-HRP of the foregoing (b) for the enzyme-labeled antibody. Normal canine sera were assayed for simultaneous and day-to-day reproducibility tests (simultaneous reproducibility test: n=8 and day-to-day reproducibility test: n=3). Excellent reproducibilities are obtained in every assay (Tables 4 and 5).

**TABLE 4**

| Test for Simultaneous Reproducibility | | |
|---|---|---|
| | Observed Value(ng/ml) | CV (%) |
| Serum 1 | 5.4 ± 0.58 | 10.7 |
| Serum 2 | 16.8 ± 1.29 | 7.7 |
| Serum 3 | 12.9 ± 0.95 | 7.4 |

**TABLE 5**

| Test for Day-to-Day Reproducibility (ng/ml) | | | | | | |
|---|---|---|---|---|---|---|
| Test Sample | 1st | 2nd | 3rd | Mean | Deviation | CV (%) |
| 1 | 21.4 | 24.9. | 23.9 | 23.4 | 1.79 | 7.7 |
| 2 | 4.2 | 4.9 | 4.4 | 4.5 | 0.38 | 8.3 |
| 3 | 9.7 | 11.3 | 11.6 | 10.9 | 1.02 | 9.4 |
| 4 | 15.6 | 18.7 | 16.7 | 17.0 | 1.54 | 9.1 |

### (h) Admixture-Recovery Test

To 10 µL of canine serum (containing canine trypsin) was added 10 µL of standard antigen sample (15 or 35 ng/mL) in advance followed by addition of 100 µL of enzyme-labeled antibody (Fab'-HRP) solution. The resultant mixture was added to the antibody-coated plate at an amount of 100 µL. The canine trypsin in test samples was quantitated in the same manner as in the foregoing (e) and each recovery rate was calculated. As a result, the admixture-recovery rate is 102%. It has been found that the assays give sufficient recovery rates (Table 6).

**TABLE 6**

| Admixture-Recovery Rate | | |
|---|---|---|
| Test Sample | Observed Value (ng/ml) | Recovery Rate (%) |
| Dog Serum | 5.52 | |
| Dog Serum + 15 ng/ml | 20.821 | 102.0 |
| Dog Serum +35 ng/ml | 41.296 | 102.2 |

When the trypsin in canine sera from dogs afflicted with various diseases was quantitated using the aforementioned assay system, it was observed that the serum trypsin level in the acute pancreatitis group was significantly higher than that in the health group while that in the exocrine pancreatic insufficiency was significantly lower.

In this instance, dogs which were tested for various kinds and as a whole diagnosed as pancreatitis or exocrine pancreatic insufficiency were quantitated for canine trypsin in the same manner as in the foregoing (e). As a result, it was shown that three pancreatitis dogs had higher levels of 40.35, 70.32 and 90.9 ng/mL while two exocrine pancreatic insufficiency dogs had lower levels of 1.35 and 0.18 ng/mL. The mean of observed trypsin values from 21 healthy dogs was 14.8± 4.71 ng/mL. It has been demonstrated that the quantitation of canine trypsin is useful in the diagnosis of pancreatitis and exocrine pancreatic insufficiency.

### Example 7

### Canine Trypsin Assay by Human Trypsin EIA

Test samples were assayed for canine trypsin, using assay reagents relying on human trypsin MAb. A canine cationic trypsin or anionic trypsin sample, prepared from canine pancreas according to Example 1, was used for canine trypsin. The assay reagent for human trypsin as used herein was an in vitro diagnostic reagent for human, "Kodazyme Trypsin M· EIA" (Hoechst Marion Roussel Ltd., Japan). The assays were conducted according to a protocol directed by Kodazyme Trypsin M· EIA as follows:

Purified canine cationic trypsin and anionic trypsin were dissolved in 0.1M phosphate buffer (pH7.5) to bring the trypsin concentration to 0.5mg/mL, respectively. Five µL each of these solutions was taken up and 0.1M phosphate buffer (pH7.5) was added to the canine trypsin solution to bring the trypsin concentration to 400ng/mL. This canine trypsin solution was dispensed at an amount of 150 µL into an Eppendorf tube to which added 0.1M phosphate buffer (pH7.5) to give canine trypsin dilutions with a series of the following trypsin concentrations: 200, 100, 50, 25, 12.5 and 6.25 ng/mL. Similarly, standard trypsin dilutions with a series of the following trypsin concentrations: 600, 400, 200, 100, 50 and 10 ng/mL, were prepared from a standard human trypsin sample enclosed in the Kodazyme Trypsin M·EIA kit. The trypsin dilutions of the respective concentrations were dispensed in aliquots of 50 µL into small test tubes followed by addition of labeled antibodies in aliquots of 300 µL.
To each tube was added one antibody-coupled ball and the mixture was incubated at 37°C for 30 min. Next, the antibody-coupled ball was washed 2 times with a washing liquid (2mL x 2). The ball was transferred into a fresh tube into which was dispensed 500 µL of substrate solution. Thereafter, the reaction was stopped by adding 3 mL of stopping solution to the tube. Duplicate assays were conducted for a single sample. Each reaction mixture was measured for absorbance at 492 nm (A₄₉₂). As a result, it has been found that neither canine cationic trypsin nor canine anionic trypsin immunoreacts with human trypsin MAb of Kodazyme Trypsin M·EIA. Thus, it is apparent that it is impossible to assay canine cationic trypsin and canine anionic trypsin with human trypsin MAb. The results are shown in Tables 7 to 9.

**TABLE 7**

| | Standard Human Trypsin Concentration (ng/ml) | | | | | | |
|---|---|---|---|---|---|---|---|
| | 0 | 10 | 50 | 100 | 200 | 400 | 600 |
| A₄₉₂ | 0.017 | 0.065 | 0.224 | 0.399 | 0.538 | 1.147 | 1.584 |
| | 0.017 | 0.131 | 0.202 | - | 0.697 | 0.953 | - |

**TABLE 8**

| | Canine Cationic Trypsin Concentration (ng/ml) | | | | | | |
|---|---|---|---|---|---|---|---|
| | 6.25 | 12.5 | 25 | 50 | 100 | 200 | 400 |
| A₄₉₂ | 0.048 | 0.021 | 0.023 | 0.017 | 0.029 | 0.017 | 0.021 |
| | 0.019 | 0.019 | 0.018 | 0.017 | 0.019 | 0.015 | 0.019 |

**TABLE 9**

| | Canine Anionic Trypsin Concentration (ng/ml) | | | | | | |
|---|---|---|---|---|---|---|---|
| | 6.25 | 12.5 | 25 | 50 | 100 | 200 | 400 |
| A₄₉₂ | 0.023 | 0.020 | 0.023 | 0.021 | 0.020. | 0.023 | 0.032 |
| | 0.023 | 0.024 | 0.021 | 0.020 | 0.022 | 0.019 | 0.038 |

### Industrial Applicability

Provided by the present invention are production of monoclonal antibodies specifically reactive to canine trypsin, detection means for trypsin, including immunohistostaining and reagents therefor. Methods for detecting or measuring trypsin, including for example EIA systems, are provided.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A monoclonal antibody against canine trypsin or a canine trypsin-related substance.

2. The monoclonal antibody according to Claim 1 which is an antibody to at least one member selected from the group consisting of canine cationic trypsinogen, canine anionic trypsinogen, canine cationic trypsin, canine anionic trypsin and canine trypsin-related substances derived therefrom.

3. The monoclonal antibody according to Claim 1 which is an antibody to at least one member selected from the group consisting of (a) a protein of SEQ ID NO: 1 or SEQ ID NO: 2 in the Sequence Listing, or a salt thereof and (b) a peptide fragment thereof, or a salt thereof.

4. The monoclonal antibody according to any of Claims 1 to 3 which is an antibody to at least one member selected from the group consisting of canine cationic trypsinogen, canine cationic trypsin, and canine cationic trypsin-related substance derived therefrom.

5. The monoclonal antibody according to any of Claims 1 to 3 which is an antibody to at least one member selected from the group consisting of
(1) a Thr¹⁶ to Ser²⁴⁷ amino acid sequence of SEQ ID NO:1 in the Sequence Listing,
(2) an Ile²⁴ to Ser²⁴⁷ amino acid sequence of SEQ ID NO:1 in the Sequence Listing,
(3) a Phe¹⁶ to Asn²⁴⁶ amino acid sequence of SEQ ID NO:2 in the Sequence Listing, and
(4) an Ile²⁴ to Asn²⁴⁶ amino acid sequence of SEQ ID NO:2 in the Sequence Listing.

6. An immunoassay for trypsin and/or trypsin-like immunoreactivity (TLI) which comprises using an assay reagent containing the monoclonal antibody according to any of Claims 1 to 5.

7. The immunoassay according to Claim 6 wherein the trypsin-like immunoreactivity (TLI) is a member selected from the group consisting of trypsinogen, trypsin and their complexes with inhibitors.

8. An immunoassay reagent for trypsin and/or trypsin-like immunoreactivity (TLI) which comprises an effective amount of a monoclonal antibody according to any of Claims 1 to 5.

9. The immunoassay reagent according to Claim 8 wherein the trypsin-like immunoreactivity (TLI) is a member selected from the group consisting of trypsinogen, trypsin and their complexes with inhibitors.


SEQUENCE LISTING

<110> Fuji Yakuhin Kogyo Kabushiki Kaisha

<120> Monoclonal Antibody against Canine Trypsin

<130> FJ-94PCT

<140>
<141>

<150> JP 10-236609
<151> 1998-08-10

<150> JP 11-63990
<151> 1999-03-10

<160> 5

<170> PatentIn Ver. 2.0

<210> 1
<211> 247
<212> PRT
<213> Dog Pancreas

<400> 1
Met Asn Pro Leu Leu Ile Leu Ala Phe Leu Gly Ala Ala Val Ala Thr
 1               5                  10                  15

Pro Thr Asp Asp Asp Asp Lys Ile Val Gly Gly Tyr Thr Cys Glu Glu
            20                  25                  30

Asn Ser Val Pro Tyr Gln Val Ser Leu Asn Ala Gly Tyr His Phe Cys
        35                  40                  45

Gly Gly Ser Leu Ile Ser Asp Gln Trp Val Val Ser Ala Ala His Cys
    50                  55                  60

Tyr Lys Ser Arg Ile Gln Val Arg Leu Gly Glu Tyr Asn Ile Asp Val
65                  70                  75                  80

Leu Glu Gly Asn Glu Gln Phe Ile Asn Ser Ala Lys Val Ile Arg His
                85                  90                  95

Pro Asn Tyr Asn Ser Trp Ile Leu Asp Asn Asp Ile Met Leu Ile Lys
            100                 105                 110

Leu Ser Ser Pro Ala Val Leu Asn Ala Arg Val Ala Thr Ile Ser Leu
        115                 120                 125

Pro Arg Ala Cys Ala Ala Pro Gly Thr Gln Cys Leu Ile Ser Gly Trp
    130                 135                 140

Gly Asn Thr Leu Ser Ser Gly Thr Asn Tyr Pro Glu Leu Leu Gln Cys
145                 150                 155                 160

Leu Asp Ala Pro Ile Leu Thr Gln Ala Gln Cys Glu Ala Ser Tyr Pro

                165                 170                 175

Gly Gln Ile Thr Glu Asn Met Ile Cys Ala Gly Phe Leu Glu Gly Gly
            180                 185                 190

Lys Asp Ser Cys Gln Gly Asp Ser Gly Gly Pro Val Val Cys Asn Gly
        195                 200                 205

Glu Leu Gln Gly Ile Val Ser Trp Gly Tyr Gly Cys Ala Gln Lys Asn
    210                 215                 220

Lys Pro Gly Val Tyr Thr Lys Val Cys Asn Phe Val Asp Trp Ile Gln
225                 230                 235                 240

Ser Thr Ile Ala Ala Asn Ser
                245

<210> 2
<211> 246
<212> PRT
<213> Dog Pancreas

<400> 2
Met Lys Thr Phe Ile Phe Leu Ala Leu Leu Gly Ala Thr Val Ala Phe
  1               5                  10                  15

Pro Ile Asp Asp Asp Asp Lys Ile Val Gly Gly Tyr Thr Cys Ser Arg
             20                  25                  30

Asn Ser Val Pro Tyr Gln Val Ser Leu Asn Ser Gly Tyr His Phe Cys
         35                  40                  45

Gly Gly Ser Leu Ile Asn Ser Gln Trp Val Val Ser Ala Ala His Cys
     50                  55                  60

Tyr Lys Ser Arg Ile Gln Val Arg Leu Gly Glu Tyr Asn Ile Ala Val
 65                  70                  75                  80

Ser Glu Gly Gly Glu Gln Phe Ile Asn Ala Ala Lys Ile Ile Arg His
                 85                  90                  95

Pro Arg Tyr Asn Ala Asn Thr Ile Asp Asn Asp Ile Met Leu Ile Lys
            100                 105                 110

Leu Ser Ser Pro Ala Thr Leu Asn Ser Arg Val Ser Ala Ile Ala Leu
        115                 120                 125

Pro Lys Ser Cys Pro Ala Ala Gly Thr Gln Cys Leu Ile Ser Gly Trp
    130                 135                 140

Gly Asn Thr Gln Ser Ile Gly Gln Asn Tyr Pro Asp Val Leu Gln Cys
145                 150                 155                 160

Leu Lys Ala Pro Ile Leu Ser Asp Ser Val Cys Arg Asn Ala Tyr Pro
                165                 170                 175

Gly Gln Ile Ser Ser Asn Met Met Cys Leu Gly Tyr Met Glu Gly Gly
            180                 185                 190

Lys Asp Ser Cys Gln Gly Asp Ser Gly Gly Pro Val Val Cys Asn Gly
        195                 200                 205

Glu Leu Gln Gly Val Val Ser Trp Gly Ala Gly Cys Ala Gln Lys Gly
    210                 215                 220

Lys Pro Gly Val Ser Pro Lys Val Cys Lys Tyr Val Ser Trp Ile Gln
225                 230                 235                 240

Gln Thr Ile Ala Ala Asn
                245

<210> 3
<211> 20
<212> PRT
<213> Artificial Sequence
<220>
<223> Description of Artificial Sequence: Designed
      peptide to act as an immunogen

<400> 3
Cys Leu Ile Ser Gly Trp Gly Asn Thr Gln Ser Ile Gly Gln Asn Tyr
  1               5                  10                  15

Pro Asp Val Leu
             20

<210> 4
<211> 20
<212> PRT
<213> Artificial Sequence
<220>
<223> Description of Artificial Sequence: Designed
      peptide to act as an immunogen

<400> 4
Ile Val Gly Gly Tyr Thr Cys Ser Arg Asn Ser Val Pro Tyr Gln Val
  1               5                  10                  15

Ser Leu Asn Ser
             20

<210> 5
<211> 20
<212> PRT
<213> Artificial Sequence
<220>
<223> Description of Artificial Sequence: Designed
      peptide to act as an immunogen

<400> 5
Leu Gln Gly Val Val Ser Trp Gly Ala Gly Cys Ala Gln Lys Gly Lys
  1               5                  10                  15

Pro Gly Val Ser
             20